(19) Europäisches Patentamt
European Patent Office
Office européen des brevets

(11) **EP 4 495 252 A1**

(12) **EUROPEAN PATENT APPLICATION**
published in accordance with Art. 153(4) EPC

(43) Date of publication:
**22.01.2025 Bulletin 2025/04**

(21) Application number: 23769893.1

(22) Date of filing: **17.03.2023**

(51) International Patent Classification (IPC):
*C12Q 1/686* (2018.01)    *C12Q 1/6876* (2018.01)
*C12N 15/11* (2006.01)

(86) International application number:
**PCT/CN2023/082033**

(87) International publication number:
**WO 2023/174391 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.03.2022 CN 202210272902**

(71) Applicant: **GUANGZHOU DEAOU GENE TECHNOLOGY CO., LTD.**
**Guangzhou, Guangdong 510700 (CN)**

(72) Inventors:
• SHEN, Hongjie
**Guangzhou, Guangdong 510700 (CN)**
• LIU, Hao
**Guangzhou, Guangdong 510700 (CN)**

(74) Representative: **Locas, Davide et al**
**Cantaluppi & Partners S.r.l.**
**Piazzetta Cappellato Pedrocchi, 18**
**35122 Padova (IT)**

Remarks:
The complete document including Reference Table(s) and the Sequence Listing(s) can be downloaded from the EPO website

(54) **METHOD FOR ENRICHMENT AND DETECTION OF LOW-ABUNDANCE MUTANT DNA**

(57)    The invention relates to a method for enriching and detecting low-abundance mutant DNA. The invention provides a wild-type amplification blocker, wherein the Tm value ($T_{m1}$) of the wild-type amplification blocker specifically binding to a wild-type template is greater than the Tm value ($T_{m2}$) of the wild-type amplification blocker specifically binding to a mutant template, and the 3' terminus of the wild-type amplification blocker has a blocking group. The invention further provides a corresponding amplification refractory mutation system and a corresponding nucleic acid detection system, wherein the amplification refractory mutation system comprises a probe (the Tm value of the probe binding to the wild-type template is $T_{m3}$, and $T_{m1} > T_{m3} > T_{m2}$) of a template competitively binding to the wild-type amplification blocker, and forward and reverse primers. According to the technical solution of the invention, the system can enrich low-abundance DNA mutations and detect multiple types of base mutations.

FIG. 1

Processed by Luminess, 75001 PARIS (FR)

**Description**

**FIELD**

The present invention relates to the technical field of biological detection and, in particular, to a method for enriching and detecting low-abundance mutant DNA.

**BACKGROUND**

DNA often undergoes random mutations during the replication process, and the mutation frequency often increases significantly in some adverse environments (such as radiation, chemical mutagens). Gene mutation is one of the important factors in biological evolution and the fundamental cause of biological genetic diversity; but gene mutation can also cause some adverse effects, such as the occurrence of cancer and drug resistance of pathogenic microorganisms. These mutations are often mixed with a large number of normal wild-type genes, and their low mutation content often brings great difficulties to clinical detection. Therefore, high-sensitivity mutation detection technology is of great significance in guiding drug use.

In recent years, the main technologies for enriching and/or detecting low-abundance mutant DNA include sequencing, digital PCR (Polymerase Chain Reaction), COLD-PCR, ARMS-PCR, WTB-PCR, denaturing high-performance liquid chromatography (dHPLC), etc. However, these methods have their own advantages and disadvantages in terms of sensitivity, accuracy, cost, time consumption, and operation.

There is a desire to develop a novel method for enriching and detecting low-abundance mutant DNA to overcome the deficiencies in the prior art in terms of sensitivity, accuracy, cost, time consumption, operation, etc.

**SUMMARY**

The present invention is intended to provide a method for enriching and detecting low-abundance mutant DNA. The technical solution provided by the present invention is obtained by optimization and improvement on the basis of WTB-PCR, so that the system can enrich low-abundance DNA mutations and detect various base mutation types, with the advantages of high sensitivity, high accuracy, low cost, low time consumption, and simple operation.

In view of this, in a first aspect, the present invention provides a wild-type amplification blocker, wherein the Tm value of the wild-type amplification blocker specifically binding to a wild-type template of a gene to be detected is denoted $T_{m1}$, the Tm value of the wild-type amplification blocker specifically binding to a mutant template of the gene to be detected is denoted $T_{m2}$, $T_{m1} > Tm_2$; and the 3' terminus of the wild-type amplification blocker has a blocking group.

According to the technical solution of the present invention, a binding position of the wild-type amplification blocker with the gene to be detected covers a mutation site of the gene to be detected. Since $T_{m1}>T_{m2}$, within a specific annealing temperature range, the wild-type amplification blocker only binds to the wild-type template, and since the wild-type amplification blocker is modified with a blocking group at its 3' terminus, extension cannot continue, thereby blocking the amplification of the wild-type template.

Further, the blocking group comprises at least one of a dideoxycytidine, a reverse dT, an amino group, a phosphate group, a spacer (e.g., spacer C3 and spacer C5) or other groups that can block the wild-type amplification blocker from acting as a primer extension.

Further, the difference between the $T_{m1}$ and the $T_{m2}$ is 5-20 °C, such as, about 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, and 20 °C.

Further, the $T_{m1}$ is 60-75 °C, such as about 60 °C, 61 °C, 62 °C, 63 °C, 64 °C, 65 °C, 66 °C, 67 °C, 68 °C, 69 °C, 70 °C, 71 °C, 72 °C, 73 °C, 74 °C, and 75 °C.

In some embodiments, the wild-type amplification blocker is fully complementary to the wild-type template of the gene to be detected.

In some embodiments, at least one, at least two, at least three, at least four, at least five, at least six, at least seven, at least eight or at least nine bases on the wild-type amplification blocker that are complementary to the gene to be detected are RNA bases.

According to the technical solution of the present invention, the difference between $T_{m1}$ and $T_{m2}$ can be further increased by replacing the bases on the wild-type amplification blocker that are complementary to the bases at the mutation position with RNA bases.

Further, the base on the wild-type amplification blocker corresponding to the base at the mutation position of the gene to be detected is located in the middle, 5' terminus or 3' terminus of the wild-type amplification blocker.

Further, the wild-type amplification blocker is an oligonucleotide chain with a length of 20-45 bp, such as 20bp, 25bp, 30bp, 35bp, 40bp, and 45bp.

In a second aspect, the present invention provides an amplification refractory mutation system, comprising a forward primer, a reverse primer, and at least one blocker-probe combination, wherein the blocker-probe combination comprises a probe and the wild-type amplification blocker of the present invention;

the forward primer and the reverse primer are used to amplify the gene to be detected, and binding positions of the forward primer and the reverse primer with the gene to be detected are respectively located upstream and downstream of the binding position of the wild-type amplification blocker with the gene to be detected;

in each blocker-probe combination, the probe and the wild-type amplification blocker competitively bind to the gene to be detected; the binding position of the probe with the gene to be detected covers the mutation site of the gene to be detected.

According to the technical solution of the present invention, the wild-type amplification blocker is located between the forward primer and the reverse primer. Compared with the technical solution in the prior art in which a wild-type amplification blocker and amplification primers competitively bind to a template, the technical solution of the present invention avoids affecting the ability of the wild-type amplification blocker to block the amplification of a wild-type allele and the efficiency of primer annealing and binding to the mutant template due to the competitive binding. Therefore, the technical solution of the present invention further improves the sensitivity of detecting the mutant allele.

In some embodiments, the amplification refractory mutation system comprises two or more blocker-probe combinations, such as two, three, four, five, six, seven, eight, nine, and ten. The number of blocker-probe combinations can be determined according to the number and position of the mutation sites of the gene to be detected and the like, so as to achieve simultaneous detection of multiple mutation sites of the gene to be detected in one detection system.

Further, the length of the probe is 15-40 bp.

Further, the probe is a fluorescent probe.

Further, the 5' terminus of the fluorescent probe is modified with a fluorescent group which is one or more of FAM, HEX, ROX, TAMRA, Texas RED, CY5, Cy3, TET, JOE, and VIC; the 3' terminus of the molecular beacon probe is modified with a quenching group which is one or more of BHQ1, BHQ2, Dabcyl, TAMRA, MGB, and ECLIPSE.

In some embodiments, the probe is a molecular beacon probe.

In some embodiments, the probe has one or two of the following modifications: peptide nucleic acid (PNA) modification and locked nucleic acid (LNA) modification.

Further, the probe and the wild-type amplification blocker have base overlap, and the number of overlapping bases is not less than 90%, 80%, 70%, 60%, 50%, 40%, 30% or 20% of the total number of bases of the probe.

Further, the Tm value of the probe specifically binding to the wild-type template of the gene to be detected is denoted $T_{m3}$, and $T_{m1}>T_{m3}>T_{m2}$.

In a certain embodiment, the difference between $T_{m1}$ and $T_{m3}$ is 5-15°C, such as, 5 °C, 6 °C, 7 °C, 8 °C, 9 °C, 10 °C, 11 °C, 12 °C, 13 °C, 14 °C, and 15 °C.

Further, the Tm values of the forward primer and the reverse primer specifically binding to the gene to be detected are denoted $T_{m4\text{-}F}$ and $T_{m4\text{-}R}$, respectively, $T_{m1}>T_{m4\text{-}F}$, $T_{m4\text{-}R}$, and further, 55 °C$\leq T_{m4\text{-}F}$, $T_{m4\text{-}R}\leq 70$ °C.

In a third aspect of the present invention, provided is a nucleic acid detection system, comprising the amplification refractory mutation system described in the second aspect of the present invention, and further comprising a PCR buffer, a DNA polymerase, and dNTPs.

Further, the PCR buffer comprises $(NH_4)_2SO_4$, $MgCl_2$, KCl, Tris-HCl, and the like.

In some embodiments, in the PCR buffer, the concentration of the $(NH_4)_2SO_4$ is 5-20 mM, such as 5 mM, 10 mM, 15 mM, and 20 mM; the concentration of the $MgCl_2$ is 2-3 mM, such as 2 mM, 2.5 mM, and 3 mm; the concentration of the KCl is 5-20 mM, such as 5 mM, 10 mM, 15 mM, and 20 mM; the concentration of the Tris-HCl is 10-30 mM, such as 10 mM, 20 mM, and 30 mM; the pH value of the Tris-HCl is 8-8.5.

Further, in the nucleic acid detection system, the final concentration of the wild-type amplification blocker is 0.02-0.5 µM, such as 0.02 µM, 0.05 µM, 0.1 µM, 0.15 µM, 0.2 µM, 0.25 µM, 0.3 µM, 0.35 µM, 0.4 µM, 0.45 µM, and 0.5 µM.

Further, in the nucleic acid detection system, the final concentration of the probe is 0.02-0.5 µM, such as 0.02 µM, 0.05 µM, 0.1 µM, 0.15 µM, 0.2 µM, 0.25 µM, 0.3 µM, 0.35 µM, 0.4 µM, 0.45 µM, and 0.5 µM.

Further, in the nucleic acid detection system, the final concentrations of the forward primer and the reverse primer are 0.02-0.5 µM, such as 0.02 µM, 0.05 µM, 0.1 µM, 0.15 µM, 0.2 µM, 0.25 µM, 0.3 µM, 0.35 µM, 0.4 µM, 0.45 µM, and 0.5 µM.

Further, in the nucleic acid detection system, the final concentration of dNTPs is 0.2-0.5 mM, such as 0.2 mM, 0.3 mM, 0.4 mM, 0.5 mM, etc.

Further, the DNA polymerase does not have 5'-3' exonuclease activity and 3'-5' endonuclease activity.

In a fourth aspect of the present invention, provided is a nucleic acid detection kit, comprising the nucleic acid detection system described in the third aspect of the present invention.

In a fifth aspect of the present invention, provided is a nucleic acid detection method, comprising the following steps: adding the template of the gene to be detected to the nucleic acid detection system described in the present invention, and performing PCR amplification and melting curve detection;

The reaction procedure for the PCR amplification comprises: denaturation at 92-98 °C for 10-30 s; first annealing at a temperature of $T_{m1}$ for 10-30 s; second annealing at a temperature of $T_{m4-F}$ or $T_{m4-R}$ for 10-30 s; extension at a temperature greater than $T_{m3}$ and less than $T_{m1}$ for 0.5-2 min; performing hese reactions for a total of 45-55 cycles.

Further, prior to the first denaturation, the PCR amplification further comprises: pre-denaturation at 92-98 °C for 1-10 min.

In some embodiments, the PCR amplification is an asymmetric PCR amplification.

Further, the reaction procedure for the melting curve detection comprises: 92-98°C, 1 min; a temperature of $T_{m1}$, 1 min; 45-50 °C, 1 min; heating to 85 °C at a heating rate of 0.01-0.05 °C/s; performing fluorescence signal collection during the heating process.

In some embodiments, fluorescence signal collection was performed every 1 °C during the heating process.

Compared with the prior art, the technical solution of the present invention has the following beneficial effects:

(1) The wild-type amplification blocker of the present invention can enrich low-abundance mutant DNA in large quantities without affecting the amplification efficiency of mutant templates, thereby improving the specificity and sensitivity of detection to meet the actual needs of clinical detection of mutant genes;

(2) The probe of the present invention can perform melting curve detection on the amplified low-abundance mutant DNA to distinguish its mutation type, and also can use fluorescent labeling groups of different wavelengths to achieve multi-channel, multi-target single-tube detection.

**BRIEF DESCRIPTION OF THE DRAWINGS**

Various other advantages and benefits will become apparent to those skilled in the art from the following detailed description of preferred embodiments. The drawings are only for the purpose of illustrating the preferred embodiments and are not to be construed as limiting. In the drawings:

FIG. 1 shows melting curve detection results of FAM channel in Example 1;
FIG. 2 shows melting curve detection results of VIC channel in Example 1;
FIG. 3 shows melting curve detection results of Texas RED channel in Example 1;
FIG. 4 shows melting curve detection results of CY5 channel in Example 1;
FIG. 5 shows melting curve detection results of FAM channel in Example 2, where the 95A site mutated to 95G;
FIG. 6 shows melting curve detection results of Texas RED in Example 2, where the 1376G site mutated to 1376T;
FIG. 7 shows melting curve detection results of Texas RED in Example 2, where the 1388G site mutated to 1388A;
FIG. 8 shows melting curve detection results of BRAF gene mutation in Example 3;
FIG. 9 shows melting curve detection results of CYP2C9-CC type detected in Texas channel in Example 4;
FIG. 10 shows melting curve detection results of CYP2C9-AA type detected in Texas channel in Example 4;
FIG. 11 shows melting curve detection results of CYP2C9-CA type detected in Texas channel in Example 4;
FIG. 12 shows melting curve detection results of VKROC1-GG type detected in CY5 channel in Example 4;
FIG. 13 shows melting curve detection results of VKROC1-AA type detected in CY5 channel in Example 4;
FIG. 14 shows melting curve detection results of VKROC1-GA type detected in CY5 channel in Example 4;

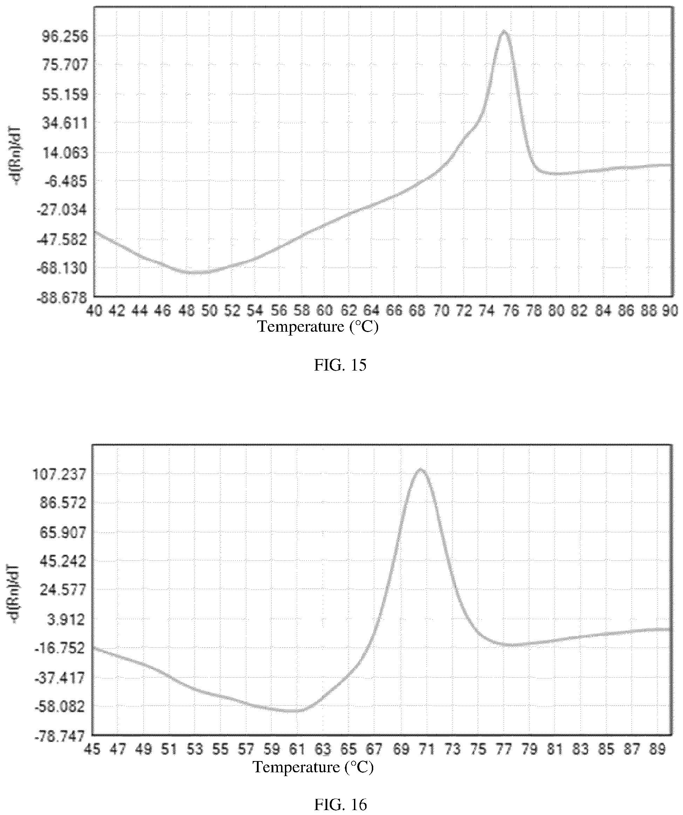

FIG. 15 shows melting curve detection results of HPV16 detected in Texas channel in Example 5;
FIG. 16 shows melting curve detection results of HPV18 detected in Texas channel in Example 5;

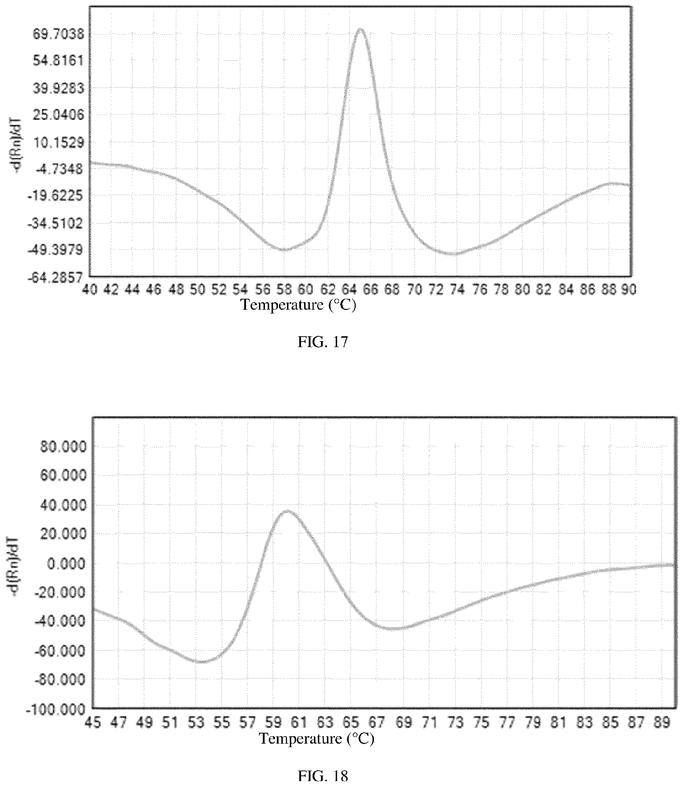

FIG. 17 shows melting curve detection results of HPV31 detected in Texas channel in Example 5;
FIG. 18 shows melting curve detection results of HPV33 detected in Texas channel in Example 5;

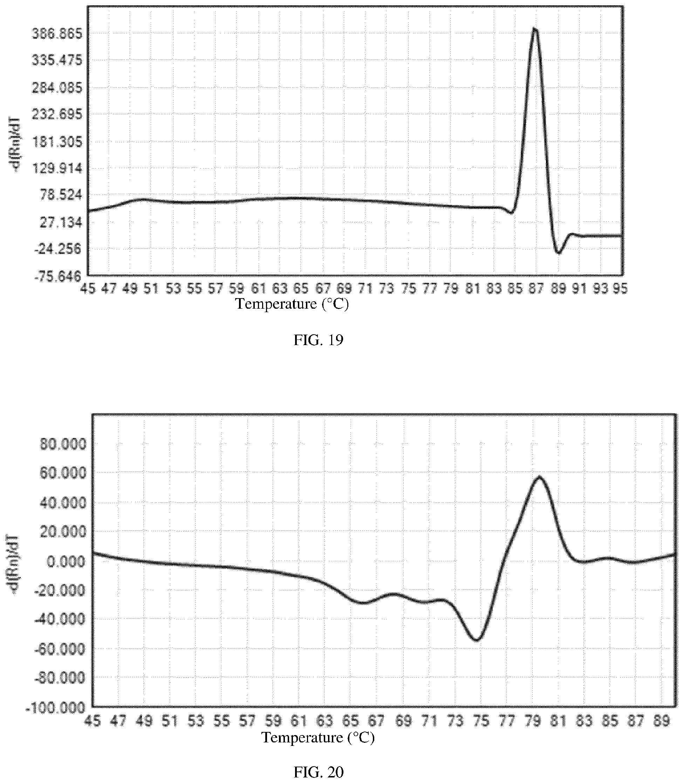

FIG. 19 shows melting curve detection results of HPV35 detected in FAM channel in Example 5;
FIG. 20 shows melting curve detection results of HPV39 detected in FAM channel in Example 5;

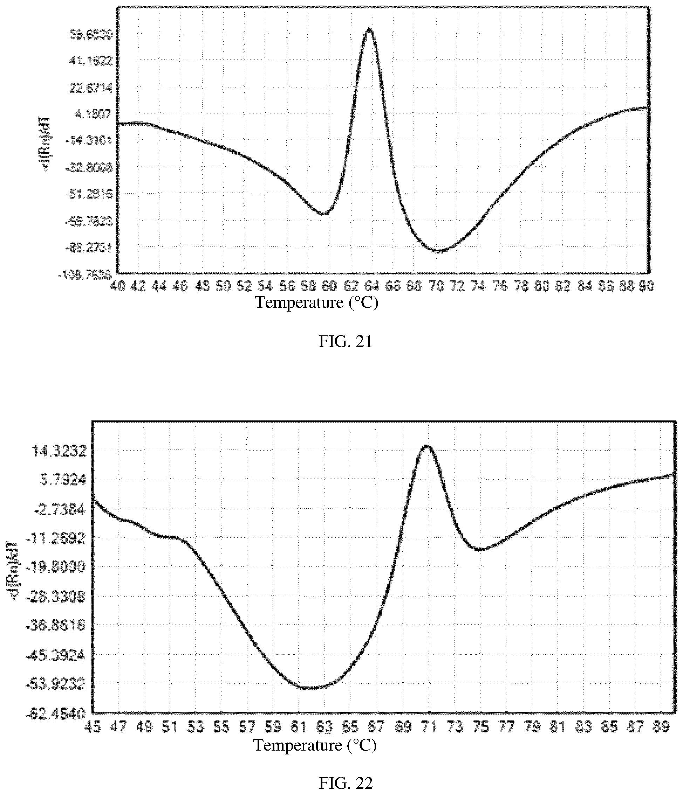

FIG. 21 shows melting curve detection results of HPV45 detected in FAM channel in Example 5;
FIG. 22 shows melting curve detection results of HPV51 detected in FAM channel in Example 5;

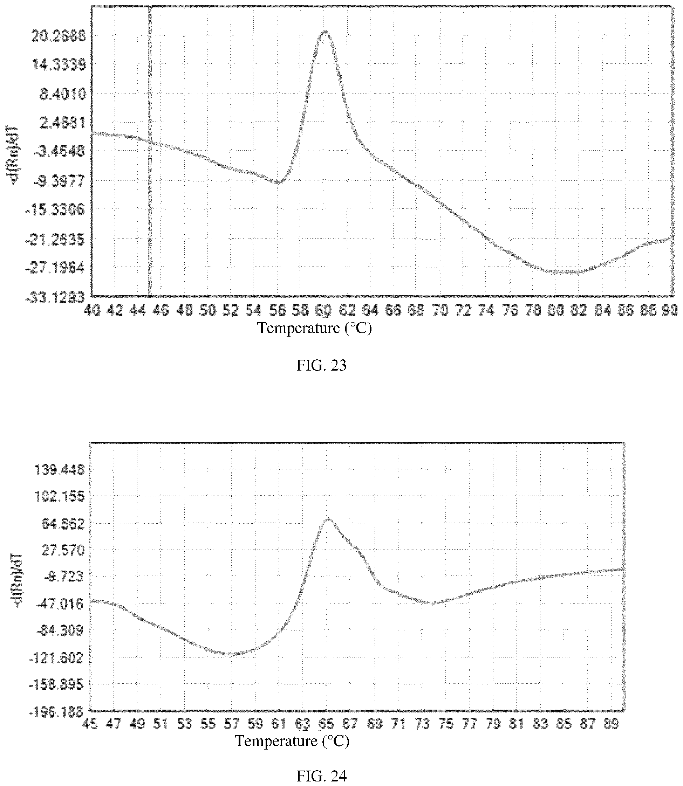

FIG. 23 shows melting curve detection results of HPV52 detected in CY5 channel in Example 5;
FIG. 24 shows melting curve detection results of HPV53 detected in CY5 channel in Example 5;

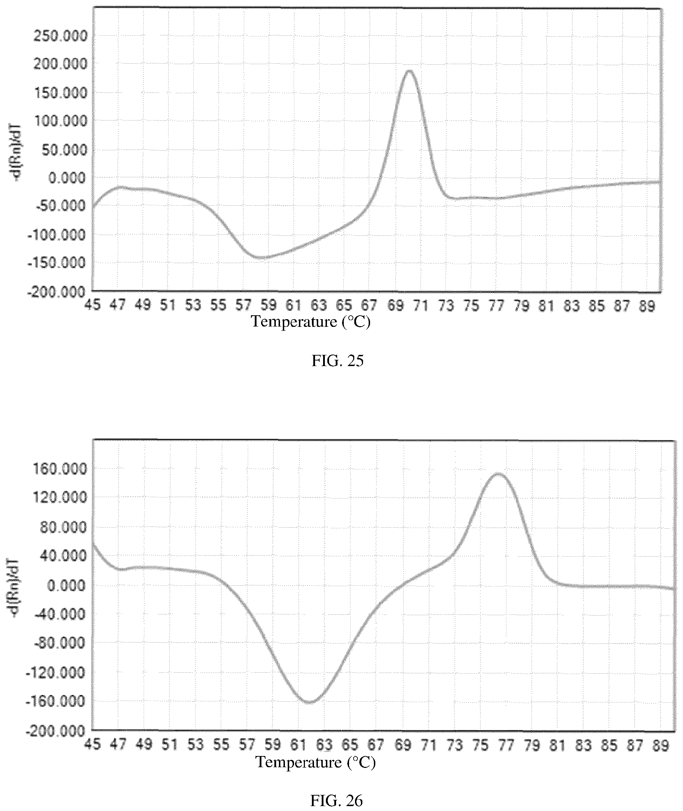

FIG. 25 shows melting curve detection results of HPV56 detected in CY5 channel in Example 5;
FIG. 26 shows melting curve detection results of HPV58 detected in CY5 channel in Example 5;

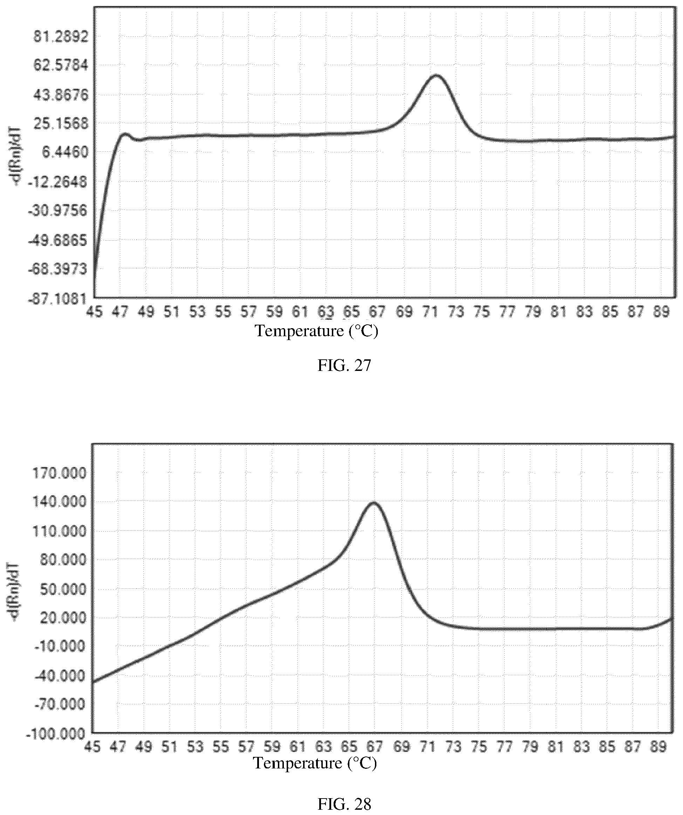

FIG. 27 shows melting curve detection results of HPV59 detected in VIC channel in Example 5;
FIG. 28 shows melting curve detection results of HPV66 detected in VIC channel in Example 5;

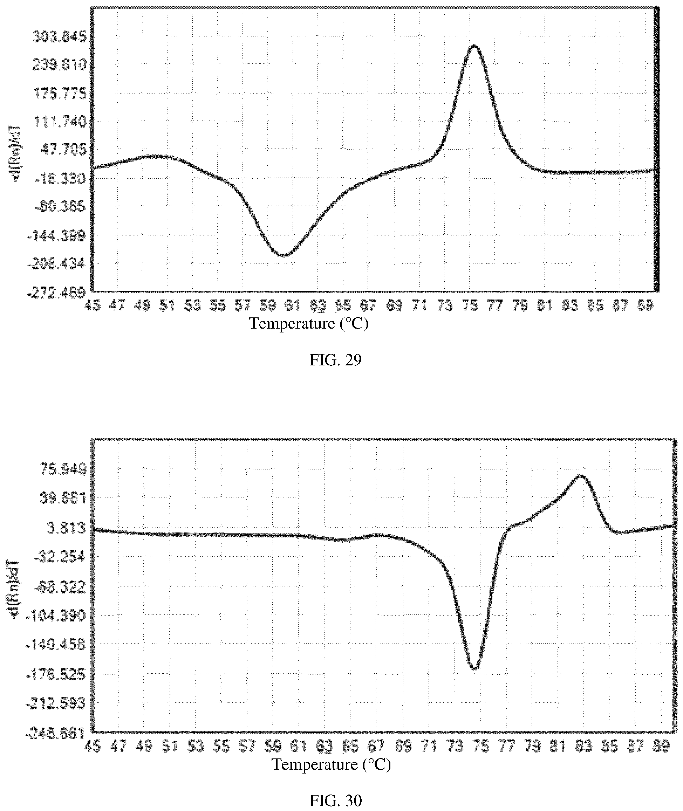

FIG. 29 shows melting curve detection results of HPV68 detected in VIC channel in Example 5;
FIG. 30 shows melting curve detection results of HPV73 detected in VIC channel in Example 5; and

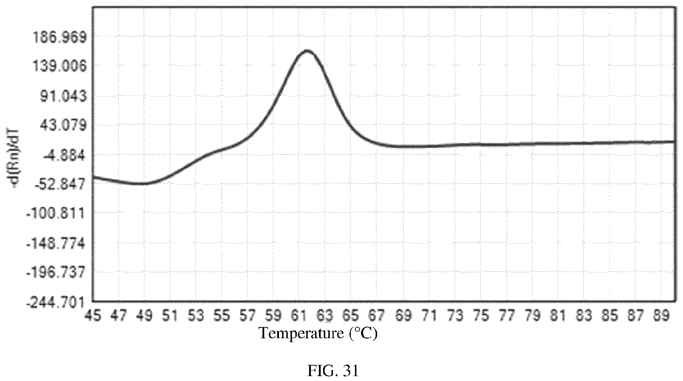

FIG. 31 shows melting curve detection results of HPV82 detected in VIC channel in Example 5.

**DESCRIPTION OF THE EMBODIMENTS**

Exemplary embodiments of the present disclosure will be described below in more detail with reference to the accompanying drawings. While the exemplary embodiments of the present disclosure are shown in the drawings, it should be understood that the present disclosure may be implemented in various forms and should not be limited to the embodiments set forth herein. Instead, these embodiments are provided so that the present disclosure will be fully understood and the scope of the present disclosure can be fully conveyed to those skilled in the art.

The present invention provides a wild-type amplification blocker (hereinafter referred to as Blocker), an amplification refractory mutation system, and a corresponding nucleic acid detection system and detection method.

The Tm value ($T_{m1}$) of the Blocker of the present invention specifically binding to a wild-type template of a gene to be detected is greater than the Tm value (Tm2) of the Blocker specifically binding to a mutant template, and the 3' terminus of the Blocker has a blocking group. In the amplification refractory mutation system of the present invention, a probe and the Blocker competitively bind to the template of the gene to be detected, and the Tm value of the probe specifically binding to the wild-type template is $T_{m3}$, wherein Tm1>Tm3>Tm2. The Tm values of a forward primer and a reverse primer specifically binding to the gene to be detected are $T_{m4-F}$ and $T_{m4-R}$, respectively, wherein $T_{m1}>T_{m4-F}$ and $T_{m1}>T_{m4-R}$.

When only the wild-type template exists in the reaction system, first annealing is performed at a temperature of $T_{m1}$, and the Blocker preferentially binds to a wild-type allele in a completely complementary manner; a second annealing is performed at a temperature of $T_{m4-F}$ or $T_{m4-R}$, and the amplification primers bind to the template to be detected in a complementary manner; extension is performed at a temperature greater than $T_{m3}$ and less than $T_{m1}$. When the amplification primers are extended to the position of the Blocker, since a polymerase used in the system does not have 5'-3' exonuclease activity, the Blocker cannot be cleaved and the extension is terminated, thereby blocking the amplification of the wild-type template.

However, in actual detection, it is found that Blocker can often only inhibit the amplification of wild-type alleles to a certain extent. When the starting amount of wild-type DNA is too high, there will be a certain amount of false positive amplification. In order to overcome this defect, the present invention also uses probes for subsequent melting curve detection. During the melting curve detection, the initial temperature is 92-98°C, so that PCR products are completely denatured into single strands. When the temperature drops to $T_{m1}$, the Blocker in the system competes with the probe and preferentially binds to possible wild-type amplification products in a completely complementary manner. Consequently, when the temperature drops later, since the wild-type amplification products that may exist in the system have been significantly reduced, the probe almost entirely binds to the mutant amplification products. This effectively reduces the interference caused by false positive amplification and improves the accuracy of melting curve detection results.

When only the mutant template exists in the reaction system, when extension is performed at a temperature greater than $T_{m3}$ and less than $T_{m1}$, neither the blocker nor the probe can bind to the template, and the amplification primers bind to the template completely complementary and extend, thereby amplifying the mutant template in large quantities.

When both the wild-type and mutant templates exist in the system, the first annealing is performed at a temperature of $T_{m1}$, and the Blocker preferentially binds to the wild-type allele in a completely complementary manner; the second annealing is performed at a temperature of $T_{m4-F}$ or $T_{m4-R}$, and the amplification primers bind to the wild-type and mutant templates in a completely complementary manner and extend at the same time; when the amplification primers extend the wild-type template to the position of the Blocker, the extension is terminated, and when the amplification primers extend the mutant template, the amplification primers can fully extend the mutant template and complete the amplification, thereby enriching low-abundance mutant DNA in large quantities.

Example 1

Amino acid mutations in codons 507-533 of the RNA polymerase β subunit encoding gene (rpoB) of Mycobacterium tuberculosis will lead to resistance to the first-line drug rifampicin (RIF). This example takes this mutation region as an example to investigate the ability of wild-type amplification blockers to detect low-abundance mutations. The specific steps are as follows:

(1) Design and synthesis of primers, probes, Blockers, and wild-type and mutant plasmids

Primer Premier6.0, DNAMAN, ClustalX and other software were used to design primers based on the core region of rifampicin resistance mutation on the rpoB gene of Mycobacterium tuberculosis, with an amplified fragment of 145bp in length; four probes were designed to completely cover the core region of the mutation. In order to block the amplification of the wild-type template, four Blockers were designed correspondingly to compete with the probes.

The forward and reverse primer sequence regions were selected to synthesize a wild-type plasmid, and the 511CCG, 516GTC, 526TAC and 531TTG mutant codons were selected to synthesize a mutant plasmid based on the clinical high-frequency mutations.

The designed primers (F-rpoB, R-rpoB), probes (P1-rpoB, P2-rpoB, P3-rpoB, P4-rpoB) and Blockers (B1-rpoB, B2-rpoB, B3-rpoB, and B4-rpoB) were all compared by BLAST for homology to ensure their specificity. The primers, probes and plasmids were synthesized by professional companies. The specific sequences are shown in Table 1.

Table 1 Sequences of primers, probes, Blockers and plasmids

| Description | Sequence (5'→3') | SEQ ID NO: |
|---|---|---|
| F-rpoB | GGCCGGTGGTCGCCGCGATCAAG | 1 |
| R-rpoB | CCGGCACGCTCACGTGACAGA | 2 |
| P1-rpoB | (FAM)-CATTGGCACCTGCCAGCTGAGCCAATG-(BHQ1) | 3 |
| P2-rpoB | (VIC)-CCTGCTCATAGACCAGAACAACCCGCTGGCAGG-(BHQ1) | 4 |
| P3-rpoB | (Texas)-CCTGCTCGGGGTAGACGCACAAGCGCGCAGG-(BHQ2) | 5 |
| P4-rpoB | (CY5)-CTGCCCGACTGTCGGCGCTGGGCAG-(BHQ3) | 6 |
| B1-rpoB | CGGCACCAGCCAGCUGAGCCAAU | 7 |
| B2-rpoB | TCATGGACCAGAACAACCCGCUG | 8 |
| B3-rpoB | TCGGGGTUGACCCACAAGCGC | 9 |
| B4-rpoB | CCGACTGTCGGCGCTGGGG | 10 |
| Wild-type plasmid (partial sequence involving the drug resistance core region) | GGAGCGGATGACCACCCAGGACGTGGAGGCGATCACACCGCAGACGTTGATCAACATCCGGCCGGTGGTCGCCGCGATCAAGGAGTTCTTCGGCACCAGCCAGCTGAGCCAATTCATGGACCAGAACAACCCGCTGTCGGGGTTGACCCACAAGCGCCGACTGTCGGCGCTGGGGCCCGGCGGTCTGTCACGTGAGCGTGCCGGGCTGGAGGTCCGCGACGTGCACCCGTCGCACTACGGCCGGATGTGCCCGATCGAAACCCCTGAGGGGCCCAACATCGGTCTGATCGGCTCGCTGTCGGT | 11 |

(continued)

| Description | Sequence (5'→3') | SEQ ID NO: |
|---|---|---|
| Mutant plasmid (partial sequence involving the drug resistance core region) | GGAGCGGATGACCACCCAGGACGTGGAGGCGATCAC ACCGCAGACGTTGATCAACATCCGGCCGGTGGTCGCC GCGATCAAGGAGTTCTTCGGCACCAGCCAGCCGAGCC AATTCATGGTCCAGAACAACCCGCTGTCGGGGTTGAC CTACAAGCGCCGACTGTTGGCGCCCGGGCCCGGCGGT CTGTCACGTGAGCGTGCCGGGCTGGAGGTCCGCGACG TGCACCCGTCGCACTACGGCCGGATGTGCCCGATCGA AACCCCTGAGGGGCCCAACATCGGTCTGATCGGCTCG CTGTCGGT | 12 |

In Table 1, the underlined elements indicate LNA-modified bases, and the highlighted elements indicate RNA bases.

The wild-type and mutant plasmids were adjusted to $10^4$ copies/$\mu$l respectively. Templates of different concentrations were prepared as follows:

10% group: 10 $\mu$l of the $10^4$ copies/$\mu$l mutant plasmid was mixed with 90 $\mu$l of the wild-type plasmid of the same concentration and a resulting mixed solution was then well shaken, thus obtaining the 10% group.
5% group: 5 $\mu$l of the $10^4$ copies/$\mu$l mutant plasmid was mixed with 95 $\mu$l of the wild-type plasmid of the same concentration and a resulting mixed solution was then well shaken, thus obtaining the 5% group.
1% group: 10 $\mu$l of the mixed solution of the 10% group was mixed with 90 $\mu$l of the $10^4$ copies/$\mu$l wild-type plasmid and a resulting mixed solution was then well shaken, thus obtaining the 1% group.
0.5% group: 10 $\mu$l of the mixed solution of the 5% group was mixed with 90 $\mu$l of the $10^4$ copies/$\mu$l wild-type plasmid and a resulting mixed solution was then well shaken, thus obtaining the 0.5% group.
0.1% group: 10 $\mu$l of the mixed solution of the 1% group was mixed with 90 $\mu$l of the $10^4$ copies/$\mu$l wild-type plasmid and a resulting mixed solution was then well shaken, thus obtaining the 0.1% group.
0.01% group: 10 $\mu$l of the mixed solution of the 0.1% group was mixed with 90 $\mu$l of the $10^4$ copies/$\mu$l wild-type plasmid and a resulting mixed solution was then well shaken, thus obtaining the 0.01% group.

Wild-type control group: $10^4$ copies/$\mu$l wild-type plasmid.

(2) Construction of real-time fluorescence PCR amplification system

A 25 $\mu$l reaction system comprising 0.2 mM of dNTPs, 10 mM of $(NH_4)_2SO_4$, 3 mM of $MgCl_2$, 10 mM of KCl, 20 mM of Tris-HCl with a pH value of 8.3, 1 U of DNA polymerase, 0.02 $\mu$M of forward primer, 0.2 $\mu$M of reverse primer, 0.1 $\mu$M of probe, 0.1 $\mu$M of Blocker, 5 $\mu$l of DNA template and the balance of water.

(3) PCR amplification and melting curve reaction

The DNA template was subjected to asymmetric PCR in the 25 $\mu$l reaction system to amplify single-stranded DNA in large quantities, and then a melting curve analysis was performed. The specific reaction procedures were as follows:
The procedure for PCR amplification was: 95 °C, 10 min; (95 °C, 15 s; 82 °C, 20 s, 61 °C, 15 s; 75 °C, 30 s, 50 cycles).
The procedure for the melting curve reaction was: 95 °C, 1 min; 82 °C, 1 min; 45 °C, 1 min, the temperature was raised to 85 °C at a rate of 0.05 °C/s, and the fluorescence signal collection was performed every 1 °C during the heating process.

Results and analysis:

The Tm value of the wild-type control detected in the FAM channel was 74.1 °C±1 °C; the Tm value of the wild-type control detected in the VIC channel was 73.5 °C±1 °C; the Tm value of the wild-type control detected in the Texas channel was 74.4 °C±1 °C; the Tm value of the wild-type control detected in the CY5 channel was 71.6 °C±1 °C. If the melting curve result of a sample to be detected differs from the Tm value of a wild-type control by at least 2 °C, the sample is determined as a mutant.

When only wild-type DNA exists in the reaction system, the Blocker and the wild-type allele are completely complementary and bound to each other, and the amplification primers bind to the DNA template to extend, and the extension is terminated when reaching the position of the Blocker, so no fluorescence signal is generated. However, in actual detection, it is found that Blocker can only inhibit the amplification of wild-type alleles to a certain extent. When the starting amount of wild-type DNA is too high, there is a certain amount of false positive amplification. As shown in the melting curve detection results, the Tm value of each fluorescent channel is consistent with the wild-type control.

When only mutant DNA exists in the reaction system, the annealing temperature (68 °C) of the Blocker and the mutant allele is lower than the extension temperature (75 °C) of the amplification primers and the DNA template. Therefore, during extension at 75 °C, the amplification primers and the mutant template extend, thereby obtaining a fluorescence signal.

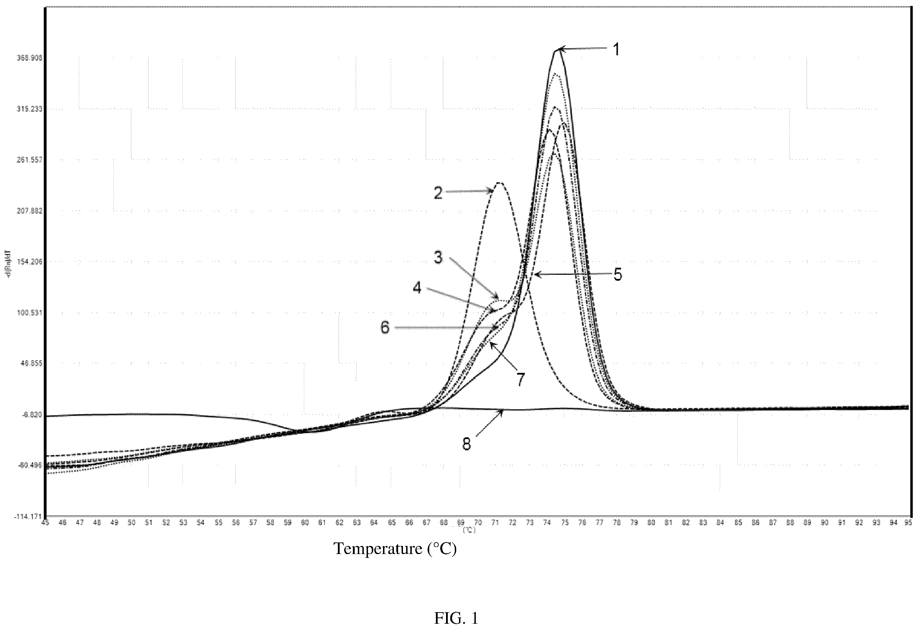

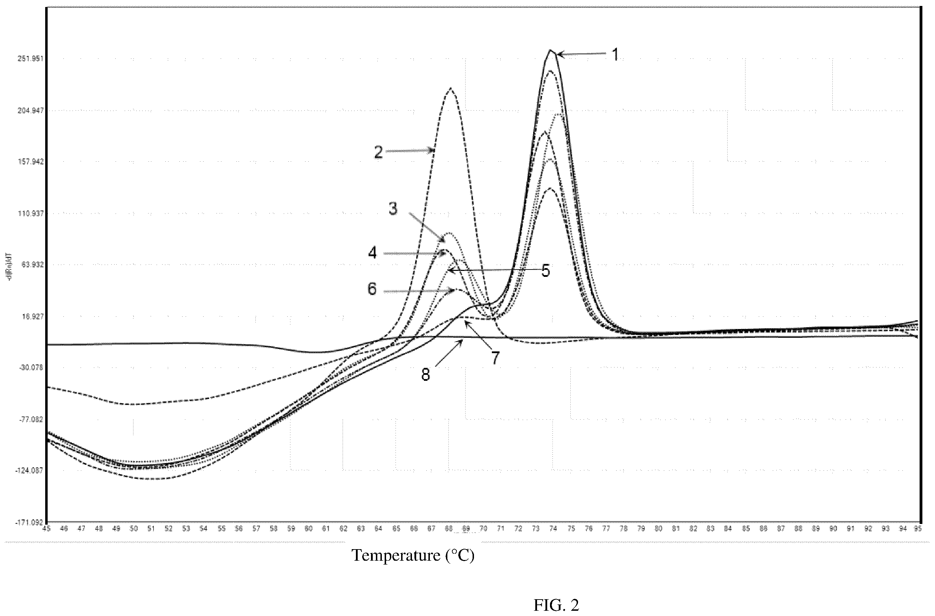

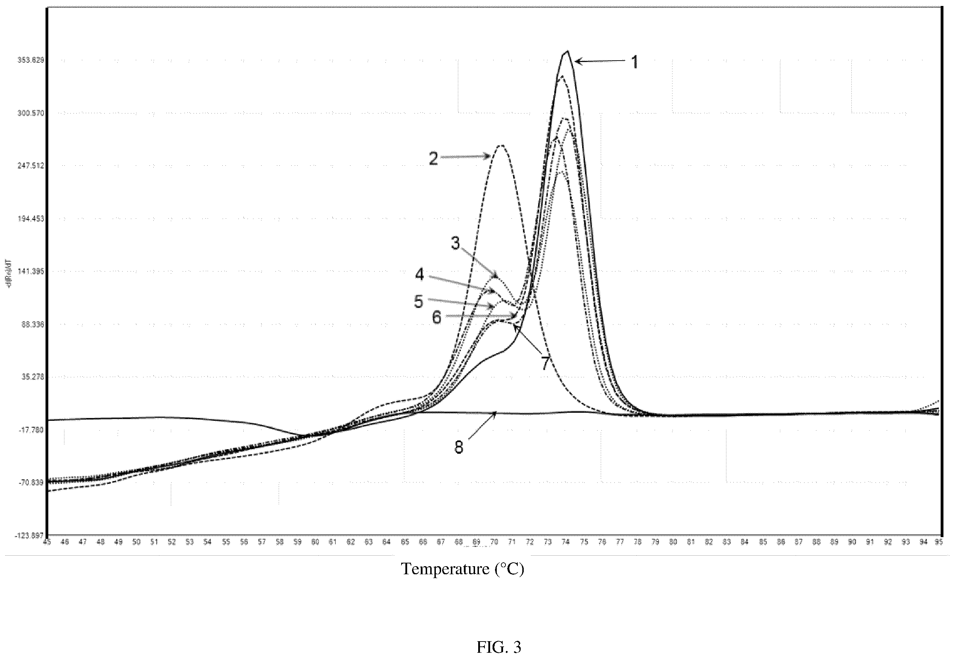

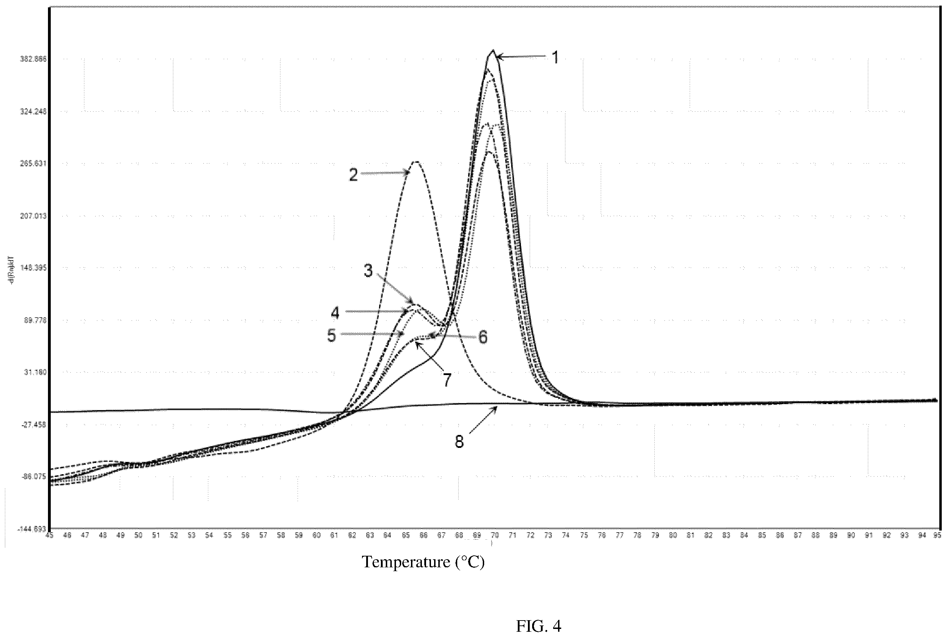

The results are shown in FIGS. 1-4. The wild-type control shows a wild-type melting peak, and the negative control shows no melting peak, indicating that the detection system can be effectively amplified with no contamination. The pure mutant only shows a mutant peak, and the 5%, 1%, 0.5%, 0.1% and 0.01% groups show both wild-type and mutant peaks. In FIGS. 1-4, 1 is the melting peak of the wild-type control, 2 is the melting peak of the 100% mutant sample, 3 is the melting peak of the mixed sample with a mutant content of 5%, 4 is the melting peak of the mixed sample with a mutant content of 1%, 5 is the melting peak of the mixed sample with a mutant content of 0.5%, 6 is the melting peak of the mixed sample with a mutant content of 0.1%, 7 is the melting peak of the mixed sample with a mutant content of 0.01 %, and 8 is the melting peak of the negative control.

Example 2

Glucose-6-phosphate dehydrogenase (G6PD) exists in various tissues and cells of the human body and participates in the pentose phosphate pathway. Glucose-6-phosphate dehydrogenase deficiency is a sex-linked incomplete dominant genetic disease. G6PD deficiency greatly increases the risk of jaundice in newborns. If not treated or treated in time, G6PD deficiency may lead to kernicterus and permanent neurological damage or hemolytic anemia. Studies have shown that mutations at different sites of the G6PD gene can lead to varying degrees of reduction in G6PD enzyme activity. 95A>G, 1376G>T, and 1388G>A mutations are the three most common types of gene mutations (accounting for 70%) in China. Currently, G6PD testing is clinically listed as one of the items for marriage examinations, prenatal examinations, and newborn examinations, so as to achieve eugenics. Since there are different ratios of mutant nucleic acids and wild-type nucleic acids in actual clinical samples, in order to better amplify low-abundance mutant nucleic acids in mixed samples, this example provides a method for detecting low-abundance G6PD mutant samples, thereby improving detection sensitivity.

In this example, the mutation of glucose-6-phosphate dehydrogenase (G6PD) gene was detected, and the specific steps were as follows:

(1) Design and synthesis of primers, probes and Blockers

Primer Premier6.0, DNAMAN, ClustalX and other software were used to design primers for the mutation regions of the G6PD gene where 95A>G and 1376G>T, 1388G>A were located, with amplified fragments of 141 bp and 150 bp in length, respectively; two probes were designed to completely cover the core region of the mutation. In order to block the amplification of the wild-type template, two blockers were designed correspondingly to compete with the probes.

Table 2 Sequences of primers, probes and Blockers

| Description | Sequence (5'→3') | SEQ ID NO: |
|---|---|---|
| F1-G6PD | GGATCCTGCGGGAAGAGC | 13 |
| R1-G6PD | GAAGGCCATCCCGGAACAG | 14 |
| F2-G6PD | GCCTCATCCTGGACGTCTTC | 15 |
| R2-G6PD | GGCCTCGGCTGCCATAAATA | 16 |

(continued)

| Description | Sequence (5'→3') | SEQ ID NO: |
|---|---|---|
| P1-G6PD | (FAM)-CGGATACACACATATTCATCATC-(BHQ1) | 17 |
| P2-G6PD | (VIC)-AATACGCCAGGCCTCACGGAG-(BHQ1) | 18 |
| B1-G6PD | TCGGATACACĊATATTCATCATCATĠĠĠ | 19 |
| B2-G6PD | AAAATAĊGCCAĠĠĊĊTCAĊGGAGCT | 20 |

In Table 2, the underlined elements indicate LNA-modified bases, and the highlighted elements indicate RNA bases.

(2) Synthesis of wild-type and mutant plasmids of G6PD gene

Since actual clinical samples are not available, artificially synthesized plasmids were used for verification. A G6PD-M wild-type plasmid was synthesized by a professional company, and the plasmid contained an original sequence with no mutations at 95A, 1376G, and 1388G, and the pUC-GW-Amp vector was used; and plasmids with mutations at 95A>G, 1376G>T and 1388G>A were synthesized separately, named as G6PD-95G-M, G6PD-1376T-M, and G6PD-1388A-M, respectively. The sequences of the G6PD-M wild-type plasmid and mutant plasmids are shown in Table 3.

Table 3 Sequences of wild type and mutant plasmids of G6PD gene

| Description | Sequence (5'→3') | SEQ ID NO: |
|---|---|---|
| G6PD-M | ATGGCAGAGCAGGTGGCCCTGAGCCGGACCCAGGTGTGCGGGATCCTGCGGGAAGAGCTTTTCCAGGGCGATGCCTTCCATCAGTCGGATACACĊCATATTCATCATCATGGGTGCATCGGGTGACCTGGCCAAGAAGAAGATCTACCCCACCATCTGGTGGCTGTTCCGGGATGGCCTTCTGCCCGAAAACACCTTCATCGTGGGCTATGCCCGTTCCCGCCTCACAGTGGCTGACATCCGCAAACAGAGTGAGCCCTTCTTCAAGGCCACCCCAGAGGAGAAGCTCTGATGACCAAGAAGCCGGGCATGTTCTTCAACCCCGAGGAGTCGGAGCTGGACCTGACCTACGGCAACAGATACAAGAACGTGAAGCTCCCTGACGĊCTATGAGCGCCTCATCCTGGACGTCTTCTGCGGGAGCCAGATGCACTTCGTGCGCAGCGACGAGCTCCĠTGAGGCCTGGCĠTATTTTCACCCCACTGCTGCACCAGATTGAGCTGGAGAAGCCCAAGCCCATCCCCTATATTTATGGCAGCCGAGGCCCCACGGAGGCAGACGAGCTGATGAAGAGA | 21 |

(continued)

| Description | Sequence (5'→3') | SEQ ID NO: |
|---|---|---|
| G6PD-95G-M | ATGGCAGAGCAGGTGGCCCTGAGCCGGACCCAGGTG TGCGGGATCCTGCGGGAAGAGCTTTTCCAGGGCGAT GCCTTCCATCAGTCGGATACAC<u>G</u>CATATTCATCATCA TGGGTGCATCGGGTGACCTGGCCAAGAAGAAGATCT ACCCCACCATCTGGTGGCTGTTCCGGGATGGCCTTCT GCCCGAAAACACCTTCATCGTGGGCTATGCCCGTTCC CGCCTCACAGTGGCTGACATCCGCAAACAGAGTGAG CCCTTCTTCAAGGCCACCCCAGAGGAGAAGCTCTGA TGACCAAGAAGCCGGGCATGTTCTTCAACCCCGAGG AGTCGGAGCTGGACCTGACCTACGGCAACAGATACA AGAACGTGAAGCTCCCTGACGCCTATGAGCGCCTCA TCCTGGACGTCTTCTGCGGGAGCCAGATGCACTTCGT GCGCAGCGACGAGCTCCGTGAGGCCTGGCGTATTTT CACCCCACTGCTGCACCAGATTGAGCTGGAGAAGCC CAAGCCCATCCCCTATATTTATGGCAGCCGAGGCCC CACGGAGGCAGACGAGCTGATGAAGAGA | 22 |
| G6PD-1376T-M | ATGGCAGAGCAGGTGGCCCTGAGCCGGACCCAGGTG TGCGGGATCCTGCGGGAAGAGCTTTTCCAGGGCGAT | 23 |

(continued)

| Description | Sequence (5'→3') | SEQ ID NO: |
|---|---|---|
| | GCCTTCCATCAGTCGGATACACACATATTCATCATCA TGGGTGCATCGGGTGACCTGGCCAAGAAGAAGATCT ACCCCACCATCTGGTGGCTGTTCCGGGATGGCCTTCT GCCCGAAAACACCTTCATCGTGGGCTATGCCCGTTCC CGCCTCACAGTGGCTGACATCCGCAAACAGAGTGAG CCCTTCTTCAAGGCCACCCCAGAGGAGAAGCTCTGA TGACCAAGAAGCCGGGCATGTTCTTCAACCCCGAGG AGTCGGAGCTGGACCTGACCTACGGCAACAGATACA AGAACGTGAAGCTCCCTGACGCCTATGAGCGCCTCA TCCTGGACGTCTTCTGCGGGAGCCAGATGCACTTCGT GCGCAGCGACGAGCTCC<u>T</u>TGAGGCCTGGCGTATTTT CACCCCACTGCTGCACCAGATTGAGCTGGAGAAGCC CAAGCCCATCCCCTATATTTATGGCAGCCGAGGCCC CACGGAGGCAGACGAGCTGATGAAGAGA | |
| G6PD-1388A-M | ATGGCAGAGCAGGTGGCCCTGAGCCGGACCCAGGTG TGCGGGATCCTGCGGGAAGAGCTTTTCCAGGGCGAT GCCTTCCATCAGTCGGATACACACATATTCATCATCA TGGGTGCATCGGGTGACCTGGCCAAGAAGAAGATCT ACCCCACCATCTGGTGGCTGTTCCGGGATGGCCTTCT GCCCGAAAACACCTTCATCGTGGGCTATGCCCGTTCC CGCCTCACAGTGGCTGACATCCGCAAACAGAGTGAG CCCTTCTTCAAGGCCACCCCAGAGGAGAAGCTCTGA TGACCAAGAAGCCGGGCATGTTCTTCAACCCCGAGG AGTCGGAGCTGGACCTGACCTACGGCAACAGATACA AGAACGTGAAGCTCCCTGACGCCTATGAGCGCCTCA TCCTGGACGTCTTCTGCGGGAGCCAGATGCACTTCGT GCGCAGCGACGAGCTCCGTGAGGCCTGGC<u>A</u>TATTTT CACCCCACTGCTGCACCAGATTGAGCTGGAGAAGCC CAAGCCCATCCCCTATATTTATGGCAGCCGAGGCCC CACGGAGGCAGACGAGCTGATGAAGAGA | 24 |

(3) Real-time fluorescence PCR amplification system

A 25 µl reaction system comprised 0.2 mM of dNTPs, 10 mM of $(NH_4)_2SO_4$, 3 mM of $MgCl_2$, 10 mM of KCl, 20 mM of Tris-HCl with a pH value of 8.3, 2 µl of DNA polymerase, 0.02 µM of forward primer, 0.2 µM of reverse primer, 0.1 µM of probe, 0.1 µM of Blocker, 5 µl of DNA template and the balance of water.

(4) Procedures for PCR amplification and melting curve reaction

The procedure for PCR amplification was: denaturation at 95 °C for 10 min; (95 °C, 15 s; 81 °C, 20 s, 63 °C, 15 s; 75 °C, 30 s, 50 cycles).
The procedure for the melting curve reaction was: 95 °C, 1 min; 81 °C, 1 min; 45 °C, 1 min, the temperature was raised to 85 °C at a rate of 0.05 °C/s, and the fluorescence signal collection was performed every 1 °C during the heating process.

(5) Analyzing the results

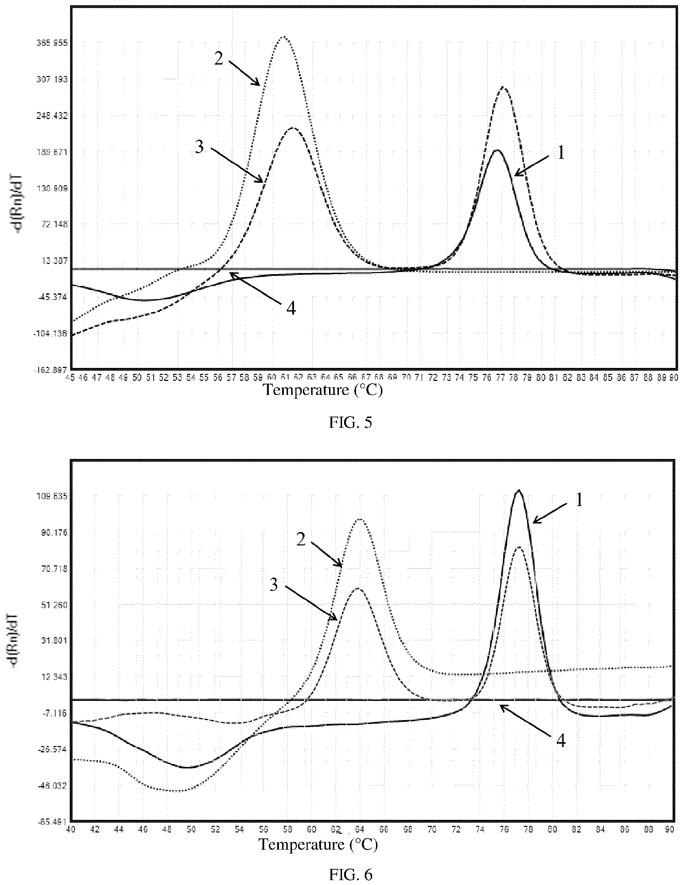

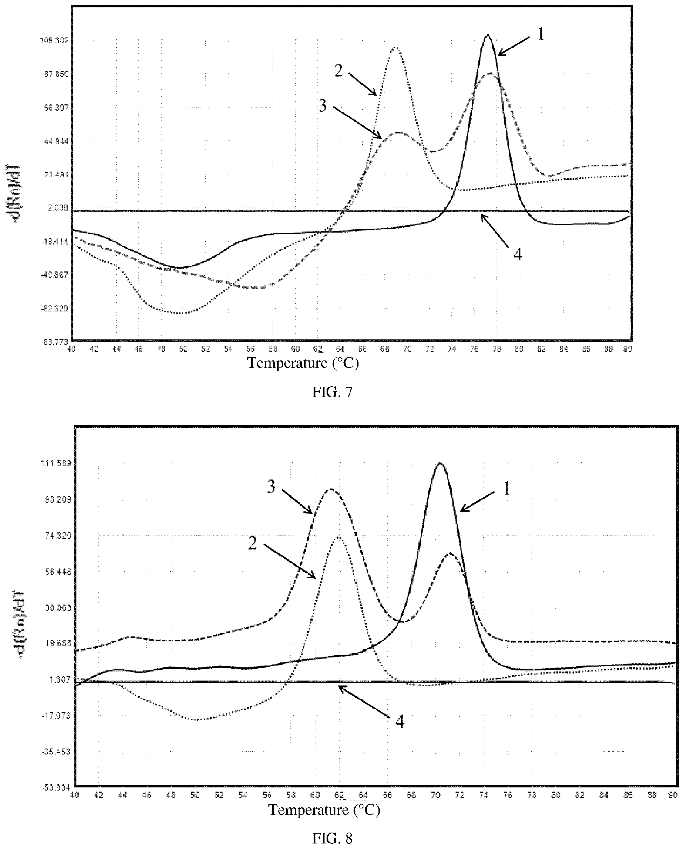

The plasmid nucleic acid with the G6PD gene 95A site mutated to 95G (G6PD-95G-M, FAM channel, FIG. 5), the plasmid nucleic acid with the G6PD gene 1376G site mutated to 1376T G6PD-1376T-M, Texas RED channel, FIG. 6), and the plasmid nucleic acid with the G6PD gene 1388G site mutated to 1388A (G6PD-1388A-M, Texas RED channel, FIG. 7), provided in Example 2, were detected, and the results are shown in FIGS. 5-7. In FIG. 5, line 1 is the melting curve of the 95A wild type, with a Tm value of 77 °C; line 2 is the melting curve of the mutant having 95A site mutated to the 95G, with a Tm value of 62 °C; line 3 is the melting curve of the mixed sample with a 95G mutant, with a content of 50%, and line 4 is the negative control. In FIG. 6, line 1 is the melting curve of the 1376G wild type, with a Tm value of 77.6 °C; line 2 is the melting curve of the 1376T mutant, with a Tm value of 64 °C; line 3 is the melting curve of the mixed sample with a 95G mutant content of 50%; and line 4 is the negative control. In FIG. 7, line 1 is the melting curve of the 1388G wild type, with a Tm value of 77.6 °C; line 2 is the melting curve of the 1388A mutant, with a Tm value of 68.5 °C, line 3 is the melting curve of the mixed sample with a 95G mutant content of 50%, and line 4 is the negative control.

Example 3

BRAF gene is an oncogene located on the long arm of chromosome 7. It contains 18 exons and can encode a B-RAF protein containing 783 amino acids. As an important transduction factor in the EGFR pathway RAS/RAF/MEK/MRK/-MAPK, the BRAF gene is involved in various physiological processes such as cell growth, differentiation and apoptosis. Studies have shown that various malignant tumors such as non-small cell lung cancer and colorectal cancer have BRAF gene mutations, among which the V600E mutation on exon 15 is the most common (about 90% or above). This mutation causes the B-RAF protein to be abnormally activated, resulting in the failure of patients to respond to treatment with EGFR-TKI drugs and EGFR monoclonal antibody drugs. As suggested in the 2010 edition of China's "Guidelines for Oncology Practice", patients with non-small cell lung cancer with normal KRAS gene test results should further examine the B-RAF gene mutation status to guide targeted drug treatment plans. During the occurrence and development of cancer, a small amount of cancer cells fall off from the primary tumor tissue and enter the blood circulation. When DNA is extracted from human peripheral blood, the proportion of mutant genes in the total DNA extracted is relatively low. Therefore, the technical solution of the present invention can achieve the enrichment of low-abundance B-RAF mutant genes and the detection of mutant bases.
In this example, the human BRAF gene mutation was detected, and the specific steps were as follows:

(1) Design and synthesis of primers, probe and Blocker

Primer Premier6.0, DNAMAN, ClustalX and other software were used to design primers for the mutation region of the BRAF gene where 1799T > A was located, with an amplified fragment of 200 bp in length; a probe was designed to completely cover the core region of the mutation. In order to block the amplification of the wild-type template, a blocker was designed correspondingly to compete with the probe.

Table 4 Sequences of primers, probe and Blocker for BRAF gene

| Description | Sequence (5'→3') | SEQ ID NO: |
| --- | --- | --- |
| F1-BRAF | ACTCTTCATAATGCTTGCTCTG | 25 |
| R1-BRAF | CCTCAATTCTTACCATCCACAA | 26 |

(continued)

| Description | Sequence (5'→3') | SEQ ID NO: |
|---|---|---|
| P1-BRAF | (FAM)-<br><br>CCACTCCATCGAGATTTCȦCTGTAGCTAGACC-<br>(BHQ1) | 27 |
| B1-BRAF | CCACTCĊȦU̇ĊGAGATTT<u>C</u>ACTGTȦĠĊTAGACC | 28 |

In Table 4, the single underlined element indicates an LNA-modified base, and the highlighted elements indicate RNA bases.

(2) Synthesis of wild-type and mutant plasmids of BRAF gene

Since actual clinical samples are not available, artificially synthesized plasmids were used for test verification. The BRAF-1799T-M wild-type plasmid and the BRAF-1799A-M mutant plasmid were synthesized by a professional company, and the pUC-GW-Amp vector was used; the sequences of the wild-type plasmid and the mutant plasmid are shown in Table 5.

Table 5 Sequences of wild type and mutant plasmids of BRAF gene

| Description | Sequence (5'→3') | SEQ ID NO: |
|---|---|---|
| BRAF-1799T-M | TATATAGGCTAAATAGAACTAATCATTGTTTTAGACATACT<br>TATTGACTCTAAGAGGAAAGATGAAGTACTATGTTTTAAAG<br>AATATTATATTACAGAATTATAGAAATTAGATCTCTTACCT<br>AAACTCTTCATAATGCTTGCTCTGATAGGAAAATGAGATCT<br>ACTGTTTTCCTTTACTTACTACACCTCAGATATATTTCTTCA<br>TGAAGACCTCACAGTAAAAATAGGTGATTTTGGTCTAGCTA<br>CAGTGAAATCTCGATGGAGTGGGTCCCATCAGTTTGAACA<br>GTTGTCTGGATCCATTTTGTGGATGGTAAGAATTGAGGCTA<br>TTTTTCCACTGATTAAATTTTTGGCCCTGAGATGCTGCTGAG<br>TTACTAGAAAGTCATTGAAGGTCTCAACTATAGTATTTTCA<br>TAGTTCCCAGTATTCACAAAAATCAGTGTTCTTATTTTTTAT<br>GTCCGAAGGCAGTTTGAACAGTTGTCTGGATCCCCTTCGG | 29 |

(continued)

| Description | Sequence (5'→3') | SEQ ID NO: |
|---|---|---|
| BRAF-1799A-M | TATATAGGCTAAATAGAACTAATCATTGTTTTAGACATACT TATTGACTCTAAGAGGAAAGATGAAGTACTATGTTTTAAAG AATATTATATTACAGAATTATAGAAATTAGATCTCTTACCT AAACTCTTCATAATGCTTGCTCTGATAGGAAAATGAGATCT ACTGTTTTCCTTTACTTACTACACCTCAGATATATTTCTTCA TGAAGACCTCACAGTAAAAATAGGTGATTTTGGTCTAGCTA CAG<u>A</u>GAAATCTCGATGGAGTGGGTCCCATCAGTTTGAACA GTTGTCTGGATCCATTTTGTGGATGGTAAGAATTGAGGCTA TTTTTCCACTGATTAAATTTTTGGCCCTGAGATGCTGCTGAG TTACTAGAAAGTCATTGAAGGTCTCAACTATAGTATTTTCA TAGTTCCCAGTATTCACAAAAATCAGTGTTCTTATTTTTAT GTCCGAAGGCAGTTTGAACAGTTGTCTGGATCCCCTTCGG | 30 |

(3) Real-time fluorescence PCR amplification system

A 25 $\mu$l reaction system comprised 0.2 mM of dNTPs, 10 mM of $(NH_4)_2SO_4$, 3 mM of $MgCl_2$, 10 mM of KCl, 20 mM of Tris-HCl with a pH value of 8.3, 2 $\mu$l of DNA polymerase, 0.02 $\mu$M of forward primer, 0.2 $\mu$M of reverse primer, 0.1 $\mu$M of probe, 0.1 $\mu$M of Blocker, 5 $\mu$l of DNA template and the balance of water.

(4) Procedures for PCR amplification and melting curve reaction

The procedure for PCR amplification was: denaturation at 95 °C for 10 min; (95 °C, 15 s; 80 °C, 20 s, 63 °C, 15 s; 70 °C, 30 s; 50 cycles).
The procedure for the melting curve reaction was: 95 °C, 1 min; 80 °C, 1 min; 45 °C, 1 min, the temperature was raised to 85 °C at a rate of 0.05 °C/s, and the fluorescence signal collection was performed every 1 °C during the heating process.

(5) Analyzing the results

The BRAF-1799T-M wild-type plasmid and BRAF-1799A-M mutant plasmid provided in this example, and a mixed sample with a mutant content of 1‰ were detected, and the results are shown in FIG. 8, where line 1 is the melting curve of the BRAF-1799T-M wild-type plasmid with a Tm value of 70 °C; line 2 is the melting curve of the BRAF-1799A-M mutant plasmid with a Tm value of 62 °C; line 3 is a mixed sample with a BRAF-1799A-M mutant content of 1‰, and line 4 is a negative control.

Example 4

Warfarin is a commonly used drug for the prevention and treatment of thromboembolic diseases in clinical practice. It is a coumarin anticoagulant. Excessive use of warfarin can cause bleeding, and its dosage depends on many factors (mainly genetics and environment). There are large individual differences in clinical efficacy and adverse reactions and it is difficult to control the dosage of warfarin. Therefore, the dosage of warfarin needs to be determined according to different individuals.
A large number of studies have shown that the polymorphism of the VKORC1 promoter (-1639G/A) and the mutation of CYP2C9 (1075A/C) are important factors affecting the use of warfarin.

Therefore, in this example, the gene mutations of CYP2C9 (rs1057910) and VKORC1 (rs9923231) were detected, and the specific steps were as follows:

Sequences of four primers, two probes and two Blockers were designed for the regions where CYP2C9 (rs1057910) and VKORC1 (rs9923231) are located. These primers could specifically amplify the two genes respectively. Probes were designed for mutants in the amplification target region. These probes could specifically bind to the target sequences of the mutants. The typing detection was performed using the different Tm values of the melting curves. In addition, in order to improve the detection sensitivity of the mutants, Blockers were designed for the wild type, thereby enriching mutant samples to a certain extent. All sequences were synthesized by professional companies.

Table 6 Sequences of primers, probes and Blockers

| Description | Sequence (5'→3') | SEQ ID NO: |
|---|---|---|
| CYP2C9-1075A/C-F | ATGCAAGACAGGAGCCACAT | 31 |
| CYP2C9-1075A/C-R | TGGGAATGAGATAGTTTCTG | 32 |
| VKORC1-RS9923231-F | AGGGATCCCTCTGGGAAGTC | 33 |
| VKORC1-RS9923231-R | CCACCTCGGCCTCCCAAAAT | 34 |
| P-CYP2C9 | (Texas)-ACGAG<u>G</u>TCCA<u>G</u>AGATACCTTGACCTTCT-(BHQ2) | 35 |
| P-VKORC1 | (CY5)-ACAACCATT<u>G</u>GCCAGGT<u>G</u>CGGTGGCT-(BHQ3) | 36 |
| B-CYP2C9 | GTGCACGAGGTCCAGAĠATACATTGACCTTCTCCC | 37 |
| B-VKORC1 | GAAAAACAACCATTGĠCCGGGTGCGGTGGCTCAC | 38 |
| CYP2C9 (1075C/C) | ATGCAAGACAGGAGCCACATGCCCTACACAGATGCTGTGGTGCACGAGGTCCAGAGATACCTTGACCTTCTCCCCACCAGCCTGCCCCATGCAGTGACCTGTGACATTAAATTCAGAAACTATCTCATTCCCA | 39 |
| CYP2C9 (1075A/A | ATGCAAGACAGGAGCCACATGCCCTACACAGATGCTGTGGTGCACGAGGTCCAGAGATACATTGACCTTCTCCCCACCAGCCTGCCCCATGCAGTGACCTGTGACATTAAATTCAGAAACTATCTCATTCCCA | 40 |
| VKORC1 (-1639G/G) | AGGGATCCCTCTGGGAAGTCAAGCAAGAGAAGACCTGAAAAACAACCATTGGCCGGGTGCGGTGGCTCACGCCTATAATCCTAGCATTTTGGGAGGCCGAGGTGG | 41 |

(continued)

| Description | Sequence (5'→3') | SEQ ID NO: |
|---|---|---|
| VKORC1 (-1639A/A) | AGGGATCCCTCTGGGAAGTCAAGCAAGAGAA GACCTGAAAAACAACCATTGGCCAGGTGCGG TGGCTCACGCCTATAATCCTAGCATTTTGGGA GGCCGAGGTGG | 42 |

In Table 6, the underlined elements indicate LNA-modified bases, and the highlighted elements indicate RNA bases.

(2) Construction of real-time fluorescence PCR amplification system

A 25 $\mu$l reaction system comprised 0.3 mM of dNTPs, 8 mM of $(NH_4)_2SO_4$, 3 mM of $MgCl_2$, 10 mM of KCl, 20 mM of Tris-HCl with a pH value of 8.3, 2 $\mu$l of DNA polymerase, 0.02 $\mu$M of forward primer, 0.2 $\mu$M of reverse primer, 0.1 $\mu$M of probe, 0.1 $\mu$M of Blocker, 5 $\mu$l of DNA template and the balance of water.

(3) PCR amplification and melting curve reaction

The DNA template was subjected to asymmetric PCR in the 25 $\mu$l reaction system to amplify single-stranded DNA in large quantities, and then a melting curve analysis was performed. The specific reaction procedures were as follows:

The procedure for PCR amplification was: 95 °C, 10 min; (95 °C, 15 s; 75 °C, 20 s, 61 °C, 15 s; 70 °C, 30 s; 50 cycles).

The procedure for the melting curve reaction was: 95 °C, 1 min; 75 °C, 1 min; 45 °C, 1 min, the temperature was raised to 90 °C at a rate of 0.05 °C/s, and the fluorescence signal collection was performed every 1 °C during the heating process.

Results and analysis:

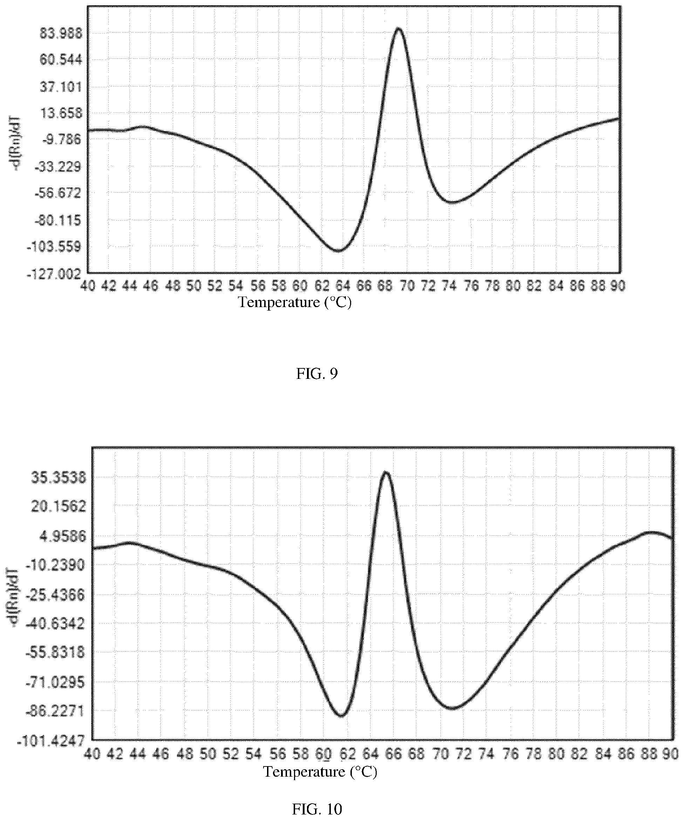

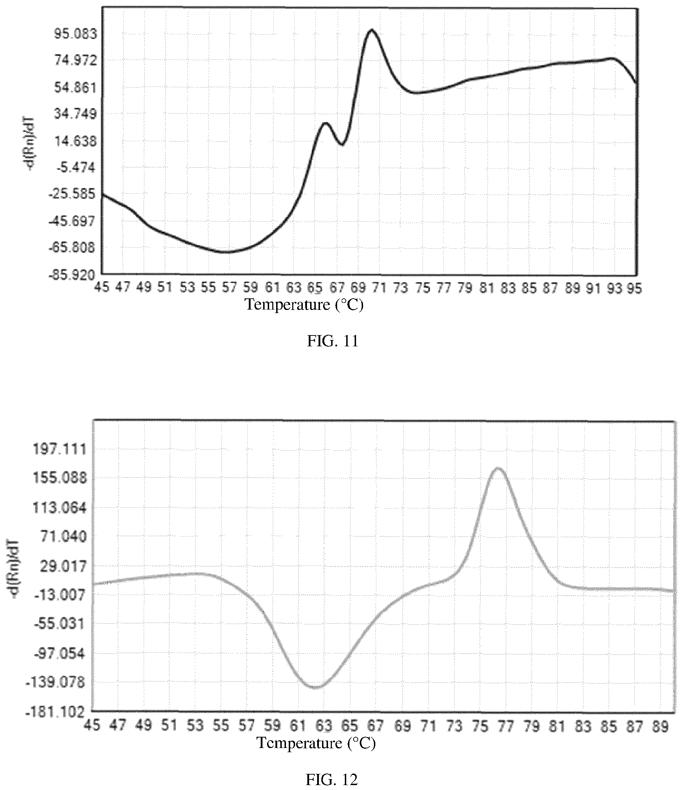

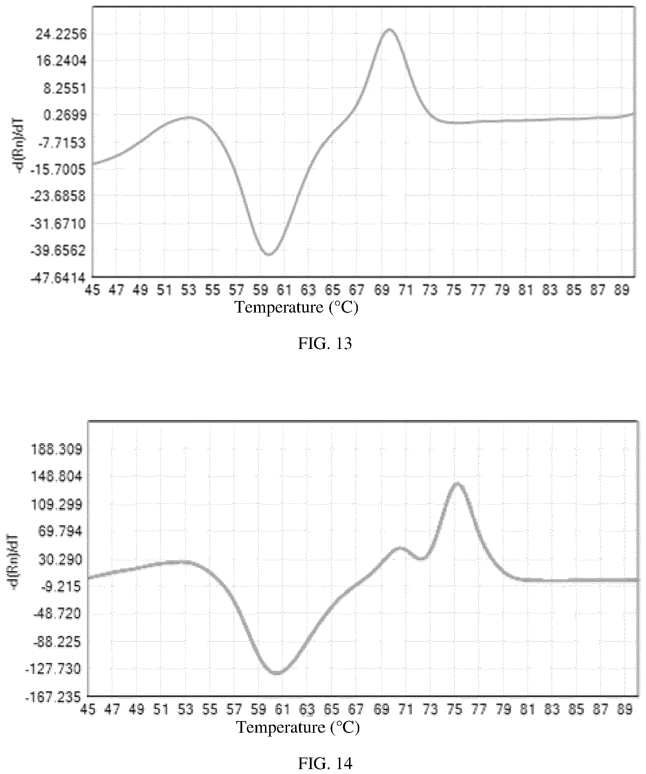

CYP2C9 was detected in the Texas channel, the Tm value of the CC type control was 69.8 °C$\pm$1 °C (FIG. 9), the Tm value of the AA type control was 65.4 °C$\pm$1 °C (FIG. 10), and the AC type control showed two peaks: a high peak and a low peak, with a Tm value of 65.6 °C$\pm$1 °C and a Tm value of 70.2 °C$\pm$1 °C, respectively (FIG. 11). VKROC1 was detected in the CY5 channel, the Tm value of the GG type control was 76.4 °C$\pm$1 °C (FIG. 12), the Tm value of the AA type control was 69.8 °C$\pm$1 °C (FIG. 13), and the GA type control showed two peaks: a high peak and a low peak, with a Tm value of 70.2 °C$\pm$1 °C and a Tm value of 75.6 °C$\pm$1 °C, respectively (FIG. 14). If the melting curve result of a sample to be detected differs from the Tm value of a control by at most 1, the sample is determined as this type.

Example 5

Human Papilloma Virus (HPV) is a double-stranded DNA virus with three regions encoded in its genome, namely the early region (E region), the late region (L region) and the long control region (LCR).

Currently, more than 100 types of HPV have been discovered, among which the common low-risk types are HPV6, HPV11, HPV42, HPV43, HPV44, etc. Low-risk types generally do not induce cancer. Common high-risk types include HPV16, HPV18, HPV31, HPV33, HPV35, HPV39, HPV49, HPV51, HPV52, HPV53, HPV56, HPV58, HPV59, HPV66, HPV68, HPV73, and HPV82. A large number of studies have shown that the occurrence of female reproductive tract malignancies such as cervical cancer is related to repeated infection with high-risk HPV viruses.

In this example, HPV type was conducted, and the specific steps are as follows:

For the more conservative L1 gene of HPV, three forward primers and one reverse primer were designed, and these primers could specifically amplify the HPV L1 gene fragment of about 150bp. 17 specific probes were designed in the amplification target region. These probes could specifically bind to the amplified fragments of HPV of the corresponding type. The typing detection was conducted using the different Tm values of the melting curves. In addition, in order to improve the detection sensitivity of low-abundance samples, five Blockers are designed for common low-risk HPV (6, 11, 42, 43, 44), thereby enriching low-abundance samples to a certain extent.

Sensitivity analysis and specificity analysis:

1. Preparation of different gradient positive standards: Plasmid powder synthesized by a professional company, was added to a marked volume of water, the nucleic acid concentration was then determined and the copy number of the plasmid was then calculated; then, gradient dilution was performed to construct plasmid standards, thus obtaining different types of plasmid standards with concentrations of $10^7$ copies/$\mu$l, $10^6$ copies/$\mu$l, $10^5$ copies/$\mu$l, $10^4$ copies/$\mu$l, $10^3$ copies/$\mu$l, $10^2$ copies/$\mu$l, 10 copies/$\mu$l and 1 copies/$\mu$l.

2. Preparation of negative control: normal healthy human genomic DNA was diluted to 10 ng/$\mu$l with a buffer containing 0.9% NaCl, 1 mmol/L EDTA, 10 mmol/L Tris-HCl and 0.1% Triton X-100, thus obtaining a negative control.

3. Preparation of low-abundance mixed samples: a mixed solution of plasmids containing 1 high-risk type and 5 low-risk types was prepared using the above diluents, and in the mixed solution, the final concentration of each high-risk plasmid was 10 copies/$\mu$l.

4. Detection of low-abundance mixed samples: The control group without blockers was used. It was found that the 10 copies/$\mu$l plasmid could not be detected in the control group without Blockers, but still could be detected in experimental groups with Blockers, indicating that the method of the present invention can improve the detection rate of low-sensitivity samples.

In this example, HPV high-risk types were identified and typed and the specific steps are as follows:

(1) Design and synthesis of primers, probes and Blockers

Primer Premier6.0, DNAMAN, ClustalX and other software were used to design universal primers based on the relatively conservative L1 gene of HPV, including three forward primers and one reverse primer, with an amplified fragment of 145 bp in length; 17 specific probes corresponding to various types were designed. In order to block the template amplification of the five common low-risk types, five Blockers were designed to compete with the probes.

The designed primers (F1-HPVL1, F2-HPVL1, F3-HPVL1, R-HPVL1), probes (P16-HPVL1, P18-HPVL1, P31-HPVL1, P33-HPVL1, P35-HPVL1, P39-HPVL1, P45-HPVL1, P51-HPVL1, P52-HPVL1, P53-HPVL1, P56-HPVL1, P58-HPVL1, P59-HPVL1, P66-HPVL1, P68-HPVL1, P73-HPVL1, P82-HPVL1) and Blockers (B6-HPVL1, B11-HPVL1, B42-HPVL1, B43-HPVL1, B44-HPVL1) were all compared by BLAST for homology to ensure their specificity. Since actual clinical samples are not available, artificially synthesized plasmids were used for verification. The primers, probes and plasmids were synthesized by professional companies. The specific sequences are shown in Table 7.

Table 7 Sequences of primers, probes and Blockers

| Description | Sequence (5'→3') | SEQ ID NO: |
|---|---|---|
| F1-HPVL1 | TTTSTTACTGTDGTDGATACHAC | 43 |
| F2- HPVL1 | TTTNTHACHGTHGTHGAYACYAC | 44 |
| F3-HPVL1 | TTTTTATTACTTGTGTTGACAC | 45 |
| R-HPVL1 | GAAAAAYAAAYTGTAADTCAWAYTC | 46 |
| P16-HPVL1 | (Texas)-TGTG<u>C</u>TGCCATAT<u>C</u>TACTT<u>C</u>AGAAACTA-(BHQ2) | 47 |
| P18-HPVL1 | (Texas)-TTCTACACAGT<u>C</u>TCCTGTAC<u>C</u>TGGG-(BHQ2) | 48 |
| P31-HPVL1 | (Texas)-TGCAATTGCAAACAGTGATACTACA-(BHQ2) | 49 |
| P33-HPVL1 | (Texas)-ACAAGTAACTAGTGACAGTACATAT-(BHQ2) | 50 |
| P35-HPVL1 | (FAM)-TG<u>C</u>TGTGT<u>C</u>TACTA<u>G</u>TGA<u>C</u>AGTACA-(BHQI) | 51 |
| P39-HPVL1 | (FAM)-TCTATAGAGT<u>C</u>TTC<u>C</u>ATAC<u>C</u>TTCTACA-(BHQI) | 52 |
| P45-HPVL1 | (FAM)-TCTACA<u>C</u>AAAATCCTGTGCCAAATAC-(BHQ1) | 53 |
| P51-HPVL1 | (FAM)-TGCCACTGCTGCGGTTTCCCCAACA-(BHQ1) | 54 |
| P52-HPVL1 | (CY5)-TGAGGTTAAAAAGGAAAGCACATAT-(BHQ3) | 55 |
| P53-HPVL1 | (CY5)-AACCACACAGTCTATGTCTACATAT-(BHQ3) | 56 |
| P56-HPVL1 | (CY5)-TGCTACAGAACAGTTAAGTAAATATG-(BHQ3) | 57 |
| P58-HPVL1 | (CY5)-TGAAGTAA<u>C</u>TAAGG<u>A</u>AGG<u>T</u>A<u>C</u>ATAT-(BHQ3) | 58 |

(continued)

| Description | Sequence (5'→3') | SEQ ID NO: |
|---|---|---|
| P59-HPVL1 | (VIC)-TTCTACTACGTCTTCTATTCCTAATG-(BHQ1) | 59 |
| P66-HPVL1 | (VIC)-AGCTAAAAGCACATTAACTAAATATGA-(BHQ1) | 60 |
| P68-HPVL1 | (VIC)-TACTACAGACTCTACTGTACCAGCT-(BHQ1) | 61 |
| P73-HPVL1 | (VIC)-AGGTACACAGGCTAGTAGCTCTACT-(BHQ1) | 62 |
| P82-HPVL1 | (VIC)-ACTGCTGCTACTCCATCAGTTGCAC-(BHQ1) | 63 |
| B6-HPVL1 | TATGTGCATĊCĠTAAĊTACATĊTTĊĊACATACACC | 64 |
| B11-HPVL1 | TATGTGCATĊTĠTGTĊTAAATCTĠĊTACATACACT | 65 |
| B42-HPVL1 | TGTGTGCCACTGCAAĊATCTĠĠTĠATACATATACA | 66 |
| B43-HPVL1 | TATGTGCCTCTACTGACCCTACTGTGCCCAGTACA | 67 |
| B44-HPVL1 | TATGTGCTGĊĊACTACACAGTĊĊĊĊTCCGTCTACA | 68 |
| HPV16 | TTTGTTACTGTTGTTGATACTACACGCAGTACAAATAT GTCATTATGTGCTGCCATATCTACTTCAGAAACTACAT ATAAAAATACTAACTTTAAGGAGTACCTACGACATGG GGAGGAATATGATTTACAGTTTATTTTTC | 69 |
| HPV18 | TTTGTTACTGTGGTAGATACCACTCCCAGTACCAATTT AACAATATGTGCTTCTACACAGTCTCCTGTACCTGGG CAATATGATGCTACCAAATTTAAGCAGTATAGCAGAC ATGTTGAGGAATATGATTTGCAGTTTATTTTTC | 70 |
| HPV31 | TTTGTTACTGTGGTAGATACCACACGCAGTACCAATA TGTCTGTTTGTGCTGCAATTGCAAACAGTGATACTAC ATTTAAAAGTAGTAATTTTAAAGAGTATTTAAGACAT GGTGAGGAATTTGATTTACAATTTATATTTC | 71 |
| HPV33 | TTTGTTACTGTGGTAGATACCACTCGCAGTACTAATAT GACTTTATGCACACAAGTAACTAGTGACAGTACATAT AAAAATGAGAATTTTAAAGAATATATAAGACATGTTG AAGAATATGATCTACAGTTTGTTTTTC | 72 |
| HPV35 | TTTGTTACTGTAGTTGATACAACCCGTAGTACAAATAT GTCTGTGTGTTCTGCTGTGTCTACTAGTGACAGTACAT ATAAAAATGACAATTTTAAGGAATATTTAAGGCATGG TGAAGAATATGATTTACAGTTTATTTTTC | 73 |

(continued)

| Description | Sequence (5'→3') | SEQ ID NO: |
|---|---|---|
| HPV39 | TTTCTTACTGTAGTGGACACTACCCGTAGTACCAACTTTACATTATCTACCTCTATAGAGTCTTCCATACCTTCTACATATGATCCTTCTAAGTTTAAGGAATATACCAGGCACGTGGAGGAGTATGATTACAATTTATATTTC | 74 |
| HPV45 | TTTGTTACTGTAGTGGACACTACCCGCAGTACTAATTTAACATTATGTGCCTCTACACAAATCCTGTGCCAAATACATATGATCCTACTAAGTTTAAGCACTATAGTAGACATGTGGAGGAATATGATTACAGTTTATTTTTC | 75 |
| HPV51 | TTTATTACCTGTGTTGATACTACCAGAAGTACAAATTTAACTATTAGCACTGCCACTGCTGCGGTTTCCCCAACATTACTCCAAGTAACTTTAAGCAATATATTAGGCATGGGGAAGAGTATGAATTGCAATTTATTTTTC | 76 |
| HPV52 | TTTGTCACAGTTGTGGATACCACTCGTAGCACTAACATGACTTTATGTGCTGAGGTTAAAAAGGAAAGCACATATAAAAATGAAAATTTTAAGGAATACCTTCGTCATGGCGAGGAATTTGATTACAATTTATTTTTC | 77 |
| HPV53 | TTTGTAACTGTTGTGGATACCACCAGGAATACAAACATGACTCTTTCCGCAACCACACAGTCTATGTCTACATATAATTCAAAGCAAATTAAACAGTATGTTAGACATGCAGAGGAATATGAATTACAATTGTGTTTC | 78 |
| HPV56 | TTTGTTACTGTAGTAGATACTACTAGAAGTACTAACATGACTATTAGTACTGCTACAGAACAGTTAAGTAAATATGATGCACGAAAAATTAATCAGTACCTTAGACATGTGGAGGAATATGAATTACAATTGTTTTTC | 79 |
| HPV58 | TTTGTTACCGTCGTTGATACCACTCGTAGCACTAATATGACATTATGCACTGAAGTAACTAAGGAAGGTACATATAAAAATGATAATTTTAAGGAATATGTACGTCATGTTGAAGAATATGACTTACAGTTTGTTTTTC | 80 |

(continued)

| Description | Sequence (5'→3') | SEQ ID NO: |
|---|---|---|
| HPV59 | TTTTTAACAGTTGTAGATACTACTCGCAGCACCAATCT TTCTGTGTGTGCTTCTACTACGTCTTCTATTCCTAATGT ATACACACCTACCAGTTTTAAAGAATATGCCAGACAT GTGGAGGAATTTGATTTGCAGTTTATATTTC | 81 |
| HPV66 | TTTGTTACTGTTGTGGATACTACCAGAAGCACCAACA TGACTATTAATGCAGCTAAAAGCACATTAACTAAATA TGATGCCCGTGAAATCAATCAATACCTTCGCCATGTG GAGGAATATGAACTACAGTTTGTGTTTC | 82 |
| HPV68 | TTTCTTACCGTTGTGGATACAACGCGCAGTACTAATTT TACATTGTCCACTACTACAGACTCTACTGTACCAGCTG TTTATGATTCTAATAAATTTAAGGAATATGTTAGGCAT GTTGAGGAATATGATTTGCAGTTTATATTTC | 83 |
| HPV73 | TTTTTAACTGTTGTAGATACTACTAGAAGCACTAATTT TTCTGTATGTGTAGGTACACAGGCTAGTAGCTCTACT ACAACGTATGCCAACTCTAATTTTAAAGAATATTTAA GACATGCAGAAGAGTATGATTTACAGTTTGTTTTTC | 84 |
| HPV82 | TTTTTATTACTTGTGTTGACACTACCAGAAGTACTAAT TTAACCATTAGCACTGCTGCTACTCCATCAGTTGCACA GACATTCACTCCAACAAACTTTAAGCAGTATATTAGG CACGGGGAAGAATATGAATTACAATTTATTTTC | 85 |

In Table 7, the underlined elements indicate LNA-modified bases, and the highlighted elements indicate RNA bases.

(2) Construction of real-time fluorescence PCR amplification system

A 25 $\mu$l reaction system comprised 0.4 mM of dNTPs, 10 mM of $(NH_4)_2SO_4$, 5 mM of $MgCl_2$, 15 mM of KCl, 20 mM of Tris-HCl with a pH value of 8.3, 3.5 $\mu$l of DNA polymerase, 0.02 $\mu$M of forward primer, 0.2 $\mu$M of reverse primer, 0.1 $\mu$M of probe, 0.1 $\mu$M of Blocker, 5 $\mu$l of DNA template and the balance of water.

(3) PCR amplification and melting curve reaction

The DNA template was subjected to asymmetric PCR in the 25 $\mu$l reaction system to amplify single-stranded DNA in large quantities, and then a melting curve analysis was performed. The specific reaction procedures were as follows:

The procedure for PCR amplification was: 95 °C, 10 min; (95 °C, 15 s; 75 °C, 20 s, 61 °C, 15 s; 70 °C, 30 s; 50 cycles).

The procedure for the melting curve reaction was: 95 °C, 1 min; 75 °C, 1 min; 45 °C, 1 min, the temperature was raised to 85 °C at a rate of 0.05 °C/s, and the fluorescence signal collection was performed every 1 °C during the heating process.

Results and analysis:

In the Texas channel, the Tm value of HPV16 control was 75.3 °C±1 °C, the Tm value of HPV18 control was 70.5 °C±1 °C, the Tm value of HPV31 control was 65.3 °C±1 °C, and the Tm value of HPV33 control was 60.2 °C±1 °C. In the FAM channel, the Tm value of HPV35 control was 86.9 °C±1 °C, the Tm value of HPV39 control was 79.5 °C±1 °C, the Tm value of HPV45 control was 63.3 °C±1 °C, and the Tm value of HPV51 control was 70.9 °C±1 °C. In the CY5 channel, the Tm value of HPV52 control was 60.4 °C±1 °C, the Tm value of HPV53 control was 65.4 °C±1 °C, the Tm value of HPV56 control was 70.1 °C±1 °C, and the Tm value of HPV58 control was 76.2 °C±1 °C. In the VIC channel, the Tm value of HPV59 control was 71.7 °C±1 °C, the Tm value of HPV66 control was 66.90 °C±1 °C, the Tm value of HPV68 control was 75.6 °C±1 °C, the Tm value of HPV73 control was 82.5 °C±1 °C, and the Tm value of HPV82 control was 61.8 °C±1 °C. If the melting curve result of a sample to be detected differs from the Tm value of a control by at most 2 °C, the sample is determined as this type of HPV.

The above description only discloses preferred embodiments of the invention, but the scope of the invention is not limited thereto, and any changes or alternatives a person skilled in the art can readily conceive of within the technical scope disclosed by the invention should be covered by the scope of the invention. Therefore, the scope of the invention should be defined by the scope of the appended claims.

**Claims**

1. A wild-type amplification blocker, wherein a Tm value of the wild-type amplification blocker specifically binding to a wild-type template of a gene to be detected is denoted $T_{m1}$, another Tm value of the wild-type amplification blocker specifically binding to a mutant template of the gene to be detected is denoted $T_{m2}$, $T_{m1} > T_{m2}$; and a 3' terminus of the wild-type amplification blocker has a blocking group.

2. The wild-type amplification blocker according to claim 1, wherein the wild-type amplification blocker is fully complementary to the wild-type template of the gene to be detected; at least one, at least two, at least three, at least four, at least five, at least six, at least seven, at least eight or at least nine bases on the wild-type amplification blocker that are complementary to the gene to be detected are RNA bases;

   the difference between the $T_{m1}$ and the $T_{m2}$ is 5-20 °C;
   the $T_{m1}$ is 65-87 °C.
   the blocking group comprises at least one of a dideoxycytidine, a reverse dT, an amino group, a phosphate group, and a spacer.

3. An amplification refractory mutation system, comprising a forward primer, a reverse primer, and at least one blocker-probe combination which comprises a probe and the wild-type amplification blocker according to claim 1;

   wherein the forward primer and the reverse primer are used to amplify the gene to be detected, and binding positions of the forward primer and the reverse primer with the gene to be detected are respectively located upstream and downstream of the binding position of the wild-type amplification blocker with the gene to be detected; and
   in each blocker-probe combination, the probe and the wild-type amplification blocker competitively bind to the gene to be detected; the binding position of the probe with the gene to be detected covers mutation sites of the gene to be detected.

4. The amplification refractory mutation system according to claim 3, wherein the probe and the wild-type amplification blocker have overlapping bases, and the number of the overlapping bases is not less than 90%, 80%, 70%, 60%, 50%, 40%, 30% or 20% of the total number of bases of the probe;

   the Tm value of the probe specifically binding to the wild-type template of the gene to be detected is denoted $T_{m3}$, and $T_{m1}>T_{m3}>T_{m2}$; and
   the difference between the $T_{m1}$ and the $T_{m3}$ is 5-15 °C.

5. The amplification refractory mutation system according to claim 3, wherein the probe is a fluorescent probe;

   the 5' terminus of the fluorescent probe is modified with a fluorescent group which is one or more of FAM, HEX, ROX, TAMRA, Texas RED, CY5, Cy3, TET, JOE, and VIC; the 3' terminus of a molecular beacon probe is modified with a quenching group which is one or more of BHQ1, BHQ2, Dabcyl, TAMRA, MGB, and ECLIPSE;
the probe is a molecular beacon probe; and
the probe has one or two of the following modifications: peptide nucleic acid (PNA) modification and locked nucleic acid (LNA) modification.

6. The amplification refractory mutation system according to claim 3, wherein the Tm values of the forward primer and the reverse primer specifically binding to the gene to be detected are denoted $T_{m4-F}$ and $T_{m4-R}$, respectively, $T_{m1} > T_{m4-F}$, $T_{m4-R}$; and

$$55\ ^{\circ}\text{C} \leq T_{m4-F},\ T_{m4-R} \leq 70\ ^{\circ}\text{C}.$$

7. A nucleic acid detection system, comprising the amplification refractory mutation system according to claim 3, and further comprising a RCR buffer, a DNA polymerase, and dNTPs.

8. The nucleic acid detection system according to claim 7, wherein the PCR buffer comprises $(NH_4)_2SO_4$, $MgCl_2$, KCl, and Tris-HCl;

in the nucleic acid detection system, a final concentration of the wild-type amplification blocker is 0.02-0.5 $\mu$M;
in the nucleic acid detection system, a final concentration of the probe is 0.02-0.5 $\mu$M;
in the nucleic acid detection system, final concentrations of the forward primer and the reverse primer are 0.02-0.5 $\mu$M;
in the nucleic acid detection system, a final concentration of the dNTPs is 0.2-0.5 mM; and
the DNA polymerase does not have 5'-3' exonuclease activity and 3'-5' endonuclease activity.

9. A nucleic acid detection kit, comprising the nucleic acid detection system according to claim 7.

10. A nucleic acid detection method, comprising the following steps: adding a template of a gene to be detected to the nucleic acid detection system according to claim 7, and performing PCR amplification and melting curve detection; wherein

a reaction procedure for the PCR amplification comprises: denaturation at 92-98 °C for 10-30 s; first annealing at a temperature of $T_{m1}$ for 10-30 s; second annealing at a temperature of $T_{m4-F}$ or $T_{m4-R}$ for 10-30 s; extension at a temperature greater than $T_{m3}$ and less than $T_{m1}$ for 0.5-2 min; performing theses reactions for a total of 45-55 cycles; and
a reaction procedure for the melting curve detection comprises: 92-98 °C for 1 min; $T_{m1}$ for 1 min; 45-50 °C for 1 min; heating to 85 °C at a heating rate of 0.01-0.05 °C/s; performing fluorescence signal collection during the heating process.

FIG. 1

EP 4 495 252 A1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

FIG. 30

FIG. 31

## INTERNATIONAL SEARCH REPORT

| | International application No. |
|---|---|
| | **PCT/CN2023/082033** |

**A. CLASSIFICATION OF SUBJECT MATTER**

C12Q 1/686(2018.01)i;C12Q 1/6876(2018.01)i;C12N 15/11(2006.01)i

According to International Patent Classification (IPC) or to both national classification and IPC

**B. FIELDS SEARCHED**

Minimum documentation searched (classification system followed by classification symbols)

C12Q, C12N

Documentation searched other than minimum documentation to the extent that such documents are included in the fields searched

Electronic data base consulted during the international search (name of data base and, where practicable, search terms used)

VEN, CNABS, CNTXT, EPTXT, USTXT, WOTXT, ISI Web of Knowledge, CNKI, EMBL: 低丰度, 广州迪澳基因科技有限公司, 扩增, 扩增阻滞物, 熔解温度, 探针, 突变, 突变DNA, 野生型阻滞扩增PCR, 引物, 阻滞扩增PCR, 阻滞物, 熔解曲线, Blocker, DNA, melting, Mutation, PCR, primer, probe, temperature, Tm, melting curve

**C. DOCUMENTS CONSIDERED TO BE RELEVANT**

| Category* | Citation of document, with indication, where appropriate, of the relevant passages | Relevant to claim No. |
|---|---|---|
| PX | CN 114592046 A (GUANGZHOU QI AUSTRALIAN GENE TECHNOLOGY CO., LTD.) 07 June 2022 (2022-06-07) <br> claims 1-10 | 1-10 |
| X | CN 107604053 A (JIN SHAOLIAN) 19 January 2018 (2018-01-19) <br> abstract, claims 1-6, 8, and 12-25, description, paragraph 0088, and embodiments 2-4 | 3, 5, 7, 9 |
| Y | CN 107604053 A (JIN SHAOLIAN) 19 January 2018 (2018-01-19) <br> abstract, claims 1-6, 8, and 12-25, description, paragraph 0088, and embodiments 2-4 | 2, 4-10 |
| X | CN 111004839 A (HANGZHOU REPUGENE TECHNOLOGY CO., LTD.) 14 April 2020 (2020-04-14) <br> abstract, claims 1-3 and 5, and description, paragraphs 0037-0047 | 1-2 |
| Y | CN 111004839 A (HANGZHOU REPUGENE TECHNOLOGY CO., LTD.) 14 April 2020 (2020-04-14) <br> abstract, claims 1-3 and 5, and description, paragraphs 0037-0047 | 2, 4-10 |
| A | CN 107034277 A (XIAMEN UNIVERSITY) 11 August 2017 (2017-08-11) <br> entire document | 1-10 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 May 2023** | **18 May 2023** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/082033**

**C. DOCUMENTS CONSIDERED TO BE RELEVANT**

| Category* | Citation of document, with indication, where appropriate, of the relevant passages | Relevant to claim No. |
|---|---|---|
| A | CN 102242211 A (WUXI RUIQI GENE BIOLOGICAL TECHNOLOGY CO., LTD.) 16 November 2011 (2011-11-16)<br>    entire document | 1-10 |
| A | CN 105420349 A (GENEFIRST LTD.) 23 March 2016 (2016-03-23)<br>    entire document | 1-10 |
| A | CN 111118119 A (HANGZHOU REPUGENE TECHNOLOGY CO., LTD.) 08 May 2020 (2020-05-08)<br>    entire document | 1-10 |
| A | WO 2013123031 A2 (VERIDEX, LLC.) 22 August 2013 (2013-08-22)<br>    entire document | 1-10 |
| A | WO 2020151283 A1 (PEKING UNIVERSITY) 30 July 2020 (2020-07-30)<br>    entire document | 1-10 |

Form PCT/ISA/210 (second sheet) (July 2022)

INTERNATIONAL SEARCH REPORT
Information on patent family members

International application No.

**PCT/CN2023/082033**

| Patent document cited in search report | | | Publication date (day/month/year) | Patent family member(s) | | | Publication date (day/month/year) |
|---|---|---|---|---|---|---|---|
| CN | 114592046 | A | 07 June 2022 | None | | | |
| CN | 107604053 | A | 19 January 2018 | None | | | |
| CN | 111004839 | A | 14 April 2020 | None | | | |
| CN | 107034277 | A | 11 August 2017 | None | | | |
| CN | 102242211 | A | 16 November 2011 | None | | | |
| CN | 105420349 | A | 23 March 2016 | None | | | |
| CN | 111118119 | A | 08 May 2020 | None | | | |
| WO | 2013123031 | A2 | 22 August 2013 | WO | 2013123031 | A3 | 12 March 2015 |
| | | | | AU | 2013221642 | A1 | 25 September 2014 |
| | | | | US | 2016130641 | A1 | 12 May 2016 |
| | | | | IL | 234021 | A0 | 30 September 2014 |
| | | | | EP | 2814985 | A2 | 24 December 2014 |
| | | | | EP | 2814985 | A4 | 30 March 2016 |
| | | | | JP | 2015517798 | A | 25 June 2015 |
| WO | 2020151283 | A1 | 30 July 2020 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Guidelines for Oncology Practice. 2010